(19)

Europäisches
Patentamt
European
Patent Office
Office européen
des brevets

(11) **EP 4 307 311 A1**

(12) **EUROPEAN PATENT APPLICATION**
published in accordance with Art. 153(4) EPC

(43) Date of publication:
**17.01.2024 Bulletin 2024/03**

(21) Application number: **22766850.6**

(22) Date of filing: **24.02.2022**

(51) International Patent Classification (IPC):
**G16H 20/00** (2018.01) **G16H 50/20** (2018.01)
**G06N 20/00** (2019.01)

(52) Cooperative Patent Classification (CPC):
**G06N 20/00; G16H 20/00; G16H 50/20**

(86) International application number:
**PCT/JP2022/007706**

(87) International publication number:
**WO 2022/190891 (15.09.2022 Gazette 2022/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.03.2021 JP 2021039643**

(71) Applicant: **Sony Group Corporation
Tokyo 108-0075 (JP)**

(72) Inventors:
• **YOSHIOKA, Shigeatsu
Tokyo 108-0075 (JP)**

• **KOBAYASHI, Yoshiyuki
Tokyo 108-0075 (JP)**
• **KUNIHIRO, Takeshi
Tokyo 108-0075 (JP)**
• **KOBAYASHI, Yohei
Tokyo 108-0075 (JP)**
• **SASADA, Shiori
Tokyo 108-0075 (JP)**
• **KATSUKI, Yugo
Tokyo 108-0075 (JP)**
• **SUZUKI, Kenji
Tokyo 108-0075 (JP)**
• **ISHII, Masato
Tokyo 108-0075 (JP)**

(74) Representative: **D Young & Co LLP
120 Holborn
London EC1N 2DY (GB)**

(54) **INFORMATION PROCESSING SYSTEM AND INFORMATION PROCESSING METHOD**

(57) It is made possible to integrate data measured by a plurality of devices and to derive an estimation result. An information processing system includes: a machine learning model learned using learning data including two or more types of modal information acquired from a patient or a subject; a setting unit (120) that sets input data to be used as input to the machine learning model from the learning data; and an output unit (100) that inputs the input data to the machine learning model and outputs an estimation result of efficacy of a medicine or a health condition for the patient or the subject, in which the setting unit sets the input data on the basis of the degree of influence given to estimation of a diagnosis result at the time of learning of the machine learning model, the degree of influence given by each piece of the modal information included in the learning data.

FIG.3

## Description

Field

The present disclosure relates to an information processing system and an information processing method.

Background

In recent years, technology is developed which assists diagnosis by a doctor or the like by outputting an estimation result of diagnosis by a learning model from a medical image such as a pathological image.

Citation List

Patent Literature

Patent Literature 1: WO 2020/174863 A

Summary

Technical Problem

Since "cancer" such as carcinoma, sarcoma, leukemia, or malignant lymphoma is developed with a very wide variety of factors being complicatedly intertwined, it is necessary to use data measured by various devices for its diagnosis. However, currently, no diagnosis assist systems are developed which are capable of integrating data measured by a plurality of devices and deriving an estimation result of diagnosis such as efficacy of a medicine or health condition.
Therefore, the present disclosure proposes an information processing system and an information processing method capable of integrating data measured by a plurality of devices and deriving an estimation result.

Solution to Problem

To solve the problems described above, an information processing system according to an embodiment of the present disclosure includes: a machine learning model learned using learning data including two or more types of modal information acquired from a patient or a subject; a setting unit that sets input data to be used as input to the machine learning model from the learning data; and an output unit that inputs the input data to the machine learning model and outputs an estimation result of efficacy of a medicine or a health condition for the patient or the subject, wherein the setting unit sets the input data on a basis of a degree of influence of each piece of the modal information included in the learning data on estimation of a diagnosis result at a time of learning of the machine learning model.

Brief Description of Drawings

FIG. 1 is a diagram illustrating a diagnosis assist system according to a first embodiment.
FIG. 2 is a block diagram illustrating a configuration example of a derivation device according to the first embodiment.
FIG. 3 is a block diagram illustrating a more detailed configuration example of a control unit according to the first embodiment.
FIG. 4 is a diagram illustrating an example of a neural network architecture for multimodal learning incorporated in a learner according to the first embodiment.
FIG. 5 is a diagram illustrating an exemplary table used in calculation of the degree of influence according to the first embodiment.
FIG. 6 is a flowchart illustrating an example of an influence degree calculating operation according to the first embodiment.
FIG. 7 is a diagram for explaining deletion and addition of modal information according to the first embodiment.
FIG. 8 is a diagram illustrating an example of a concept visualizing the degree of influence for every piece of modal information according to the first embodiment.
FIG. 9 is a diagram illustrating an example of a concept in which the degree of influence of other modal information regarding specific modal information is visualized according to the first embodiment.
FIG. 10 is a diagram illustrating an example of a concept obtained by visualizing affected regions in the modal information according to the first embodiment.

FIG. 11 is a diagram illustrating an example of a concept in which meta-information affecting in the modal information is visualized according to the first embodiment.

FIG. 12 is a diagram illustrating an example of a neural network architecture for multimodal learning incorporated in a learner according to a modification of the first embodiment.

FIG. 13 is a diagram for explaining a specific example of a flow for narrowing down a region having affected output from each piece of modal information according to the first embodiment.

FIG. 14 is a diagram illustrating an example of sensor data (modal information) acquired by a plurality of wearable devices.

FIG. 15 is a diagram for explaining a specific example of a flow from multimodal learning to derivation of an estimation result according to the first embodiment (part 1) .

FIG. 16 is a diagram for explaining a specific example of a flow from multimodal learning to derivation of an estimation result according to the first embodiment (part 2) .

FIG. 17 is a diagram illustrating an example of a neural network architecture for multimodal learning incorporated in a learner according to a second embodiment.

FIG. 18 is a schematic diagram illustrating an example of medical setting into which a diagnosis assist system according to an embodiment of the present disclosure is introduced.

FIG. 19 is a schematic diagram illustrating another example of medical setting into which a diagnosis assist system according to an embodiment of the present disclosure is introduced.

FIG. 20 is a diagram for explaining one of objects of the technology according to the present disclosure.

FIG. 21 is a diagram for explaining an application example of the technology according to the present disclosure to the precision medicine field.

FIG. 22 is a diagram for explaining an application example of the technology according to the present disclosure to the ICU monitoring field.

FIG. 23 is a diagram for explaining a specific example of a case where the technology according to the present disclosure is applied to the ICU monitoring field.

FIG. 24 is a diagram for explaining an application example of the technology according to the present disclosure to the field of operating room efficiency improvement.

FIG. 25 is a diagram for explaining a specific example in a case where the technology according to the present disclosure is applied to the field of operating room efficiency improvement.

FIG. 26 is a diagram for explaining an application example of the technology according to the present disclosure to the field of sports training.

FIG. 27 is a diagram for explaining an application example of the technology according to the present disclosure to the smart agriculture field.

FIG. 28 is a hardware configuration diagram illustrating an example of a computer that implements the technology according to the present disclosure.

FIG. 29 is a diagram schematically illustrating an overall configuration of a microscope system.

FIG. 30 is a diagram illustrating an example of an imaging method.

FIG. 31 is a diagram illustrating an example of an imaging method.

Description of Embodiments

Hereinafter, embodiments of the present disclosure will be described in detail on the basis of the drawings. Note that in each of the following embodiments, the same parts are denoted by the same symbols, and redundant description will be omitted.

The present disclosure will be described in the following order of items.

0. Introduction
0.1 Definition of Terms
1. First Embodiment
1.1 System Configuration
1.2 Derivation Device
1.3 Control Unit
1.4 Neural Network Architecture for Multimodal Learning
1.5 Method for Calculating Degree of Influence (Contribution Ratio) of Each Piece of Modal Information on Output
1.5.1 Management Table
1.5.2 Flowchart
1.5.3 Another Example of Influence Degree (Contribution Ratio) Calculating Method

1.6 About Deletion And Addition of Modal Information

1.7 Visualization of Degree of Influence of Each Piece of Modal Information

1.8 Visualization of Affected Region in Modal Information

1.8.1 About Narrowing Down Affected Region in Modal Information

1.9 Utilization of Wearable Devices

1.10 Specific Example of Flow from Multimodal Learning to Derivation of Estimation Result

1.11 Summary

2. Second Embodiment

3. Third Embodiment

4. Fourth Embodiment

4.1 Other Application Examples in Medical Field

4.1.1 Exemplary Application to Precision Medicine Field

4.1.2 Exemplary Application to ICU Monitoring Field

4.1.3 Exemplary Application to Field of Operating Room Efficiency Improvement

4.2 Exemplary Application to Fields Other Than Medical Field

4.2.1 Exemplary Application to Sports Training Field

4.2.2 Exemplary Application to Smart Agriculture Field

5. Hardware Configuration

6. Configuration Example of Microscope System of Present Disclosure

0. Introduction

"Cancer" such as carcinoma, sarcoma, leukemia, or malignant lymphoma is a disease caused by a genetic damage. When abnormal cells, whose genetic damage ranges to the function of repair and apoptosis (programmed cell death), get around the immune mechanism and repeat cell division, a mass of the abnormal cells become recognizable as "cancer".

Currently, definite diagnosis of "cancer" is performed by pathological diagnosis in which cell shapes or tissue disturbance are observed with a microscope.

However, in pathological diagnosis, it is not known how "cancer" can be stopped from growing or eliminated since in pathological diagnosis observation is made on spatially biased chromosomes in which transcriptional expression is activated due to gene abnormality or abnormality in the appearance of cells or tissues secondarily appearing due to lack of a filament component constituting a cytoskeleton.

In order to effectively treat "cancer" with a medicine, it is necessary to grasp the cause for cancer cells to continuously grow or not being eliminated by the immune mechanism. For example, in a case where the molecule of the "signaling pathway" has changed by genetic mutation and is abnormally growing, it can be treated by stopping the signaling pathway with a medicine targeting the molecule, meanwhile, in a case where T cell activation has been suppressed by an immune checkpoint molecule from a cancer cell, it can be treated by blocking the transmission of a suppression signal with an antibody medicine that inhibits the binding of the immune checkpoint.

However, since "cancer" is caused by a very wide variety of factors complicatedly intertwined, it is necessary to collect data using various devices in order to grasp the cause. Some devices can directly observe the cause, whereas other devices indirectly observe the cause through a surrogate marker correlated with the cause. In addition, pieces of data obtained by observing the phenomenon called "cancer" by different means are also complicatedly related to each other. Therefore, diagnosis assist systems that assist pathological diagnosis using machine learning are expected to support multimodal learning capable of integrating and learning data of various devices.

Therefore, in the following embodiments, it is made possible to integrate data measured by a plurality of devices to derive an estimation result of diagnosis such as efficacy of a medicine or health condition. Furthermore, in the following embodiments, it is made possible to present to a user which device is more suitable for acquiring data for specifying a medicine optimal for treatment by multimodal learning. Note that it goes without saying that the technology according to the present disclosure is not limited to diagnosis and treatment of "cancer" and can be applied to diagnosis of diseases and various estimations and responses other than diagnosis of diseases. In addition, the "diagnosis" in the following description can include various diagnoses such as a medical checkup diagnosis for confirming the health condition of a patient, a subject, or the like and a companion diagnosis for confirming the efficacy of a medicine prescribed to a patient, a subject, or the like. Furthermore, the "diagnosis" in the following description can include not only a diagnosis for medical purposes for a patient, a subject, or the like but also various diagnoses such as a diagnosis for research and development purposes.

0.1 Definition of Terms

Herein, definitions of some of the terms used in the present disclosure will be described.

In the present disclosure, the "information acquiring device" may be, for example, a medical device, a test device, a biological sample analysis device, a wearable terminal carried by a patient or a subject, or the like and may be, for example, a digital pathology scanner using digital pathology technology, next generation sequencing (NGS), a protein measuring device, computed tomography (CT)/magnetic resonance imaging (MRI)/positron emission tomography (PET), enzyme-linked immunosorbent assay (ELISA), a microarray, a wearable device, a microscope, an endoscope, or the like. Furthermore, in fields other than the medical care, the "information acquiring device" may be, for example, various sensors of any type and form, such as a camera (including color, monochrome, infrared, and others), a time of flight (ToF) sensor, an event-based vision sensor (EVS), a light detection and ranging or laser imaging detection and ranging (LIDAR), a millimeter-wave radar, an ultrasonic sensor, a proximity sensor, an inertial measurement unit (IMU), an acceleration/angular velocity/angular acceleration sensor, a potentiometer, a thermometer, a hygrometer, or a vibrometer and is not limited to those listed above.

"Modal information" may be information acquired by an information acquiring device as the above.

The "multimodal information" may be a set of modal information acquired individually by two or more information acquiring devices. In this case, the multimodal information may include two or more pieces of modal information acquired by information acquiring devices of the same type (such as cameras of the same model).

The "multimodal learning" may be learning (also referred to as training) a learning model or relearning a learned model using one or more pieces of modal information (also referred to as a data set) included in multimodal information as training data (also referred to as learning data). The data set may include two or more pieces of modal information acquired by information acquiring devices of the same type (such as cameras of the same model).

1. First Embodiment

1.1 System Configuration

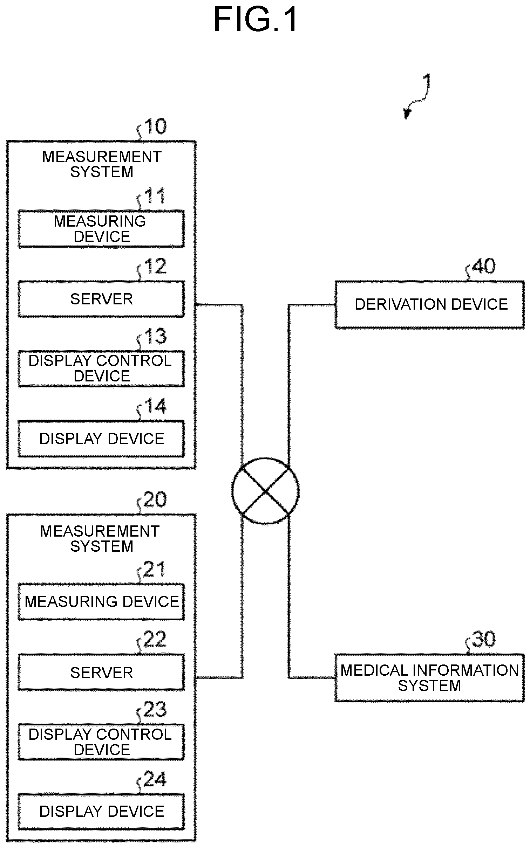

First, a diagnosis assist system (information processing system or information processing device) according to a first embodiment will be described with reference to FIG. 1. FIG. 1 is a diagram illustrating the diagnosis assist system according to the embodiment. As illustrated in FIG. 1, the diagnosis assist system 1 includes a measurement system 10, a measurement system 20, a medical information system 30, and a derivation device 40.

(Measurement System 10/20)

The measurement system 10 is a system mainly used by doctors, and is applied to, for example, a laboratory or a hospital. As illustrated in FIG. 1, the measurement system 10 includes a measuring device 11, a server 12, a display control device 13, and a display device 14.

The measuring device 11 may be, for example, a digital pathology scanner, next generation sequencing (NGS), a protein measuring device, computed tomography (CT)/magnetic resonance imaging (MRI)/positron emission tomography (PET), enzyme-linked immunosorbent assay (ELISA), a microarray, a wearable device, a microscope, an endoscope, or one or more medical devices or information processing devices that acquire various types of information (hereinafter, also referred to as measurement information) regarding a patient, an affected area thereof, or the like.

The server 12 assists a user such as a doctor or a pathologist in diagnosis in the measurement system 10 and executes retention, management, and others of the measurement information acquired by the measuring device 11. Note that the measurement information acquired by the measuring device 11 may be stored in, for example, a storage unit or the like included in the server 12 or connected to the server.

The display control device 13 receives, from the user, a request for browsing various types of information such as an electronic medical record, a diagnosis result, or a diagnosis estimation result regarding a patient, and sends the browsing request, which has been accepted, to the server 12. Then, the display control device 13 controls the display device 14 to display the various types of information received from the server 12 in response to the browsing request.

The display device 14 has a screen adopting, for example, liquid crystal, electro-luminescence (EL), a cathode ray tube (CRT), or others. The display device 14 may support 4K or 8K or may include a plurality of display devices. The display device 14 displays various types of information to a user in accordance with control from the display control device 13.

The measurement system 20 is a system applied to a hospital different from that of the measurement system 10. For example, similarly to the measurement system 10, the measurement system 20 may include a measuring device 21, a server 22, a display control device 23, and a display device 24. In that case, since each component included in the measurement system 20 may be similar to that of the measurement system 10, description thereof will be omitted.

(Medical Information System 30)

The medical information system 30 is a so-called electronic medical record system and executes retention, management, and others of information regarding diagnosis performed on a patient by a doctor, a pathologist, or the like at present or in the past, information such as a patient's medical history (hereinafter, also referred to as diagnostic information), and the like. Note that the information regarding diagnosis may include, for example, information for identifying a patient, disease information of the patient, test information and image information used for the diagnosis, the diagnosis result, prescribed medicine, and others.

(Derivation Device 40)

The derivation device 40 acquires measurement information accumulated every day in the server 12 of the measurement system 10 and/or the server 22 of the measurement system 20. The derivation device 40 also acquires diagnostic information accumulated every day in the medical information system 30. The derivation device 40 trains a learning model using the collected measurement information and diagnostic information as training data, thereby generating a learned model for estimating a diagnosis result of the patient on the basis of at least one piece of information included in the measurement information and the diagnostic information.
Note that the number of measurement systems included in the diagnosis assist system 1 may be three or more. In this case, the derivation device 40 may collect the measurement information accumulated in each of the measurement systems and train the learning model using the collected measurement information and diagnostic information as training data. Also, in the above example, the medical information system 30 may be incorporated into the measurement system 10 and/or 20. In that case, the diagnostic information may be stored in the server 12 and/or 22.
Furthermore, the derivation device 40 according to the present embodiment may be implemented by a server, a cloud server, or the like disposed on a network or may be implemented by a server 12/22 disposed in the measurement system 10/20. Alternatively, the derivation device 40 may be implemented by being disposed in a distributed manner on a system configured via a network in a manner such as that a part of the derivation device 40 is implemented by a server, a cloud server, or the like disposed on a network and that the remaining part is implemented by the server 12/22 of the measurement system 10/20.

1.2 Derivation Device

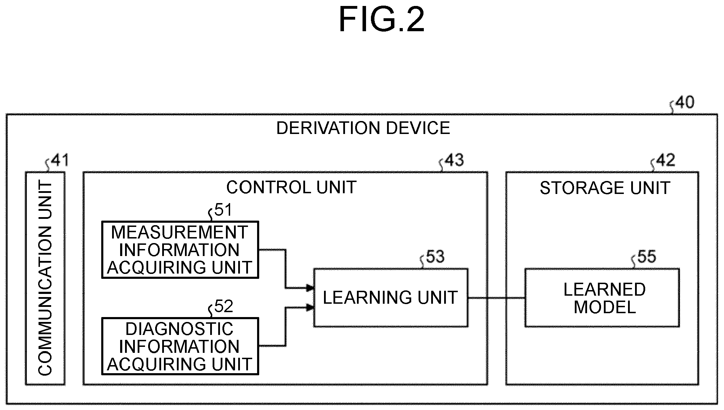

Next, a configuration example of the derivation device 40 according to the present embodiment will be described. FIG. 2 is a block diagram illustrating a configuration example of the derivation device according to the embodiment. As illustrated in FIG. 2, the derivation device 40 includes a communication unit 41, a storage unit 42, and a control unit 43.
The communication unit 41 is implemented by, for example, a network interface card (NIC) or the like. The communication unit 41 is connected to a network (not illustrated) in a wired or wireless manner and transmits and receives information to and from the measurement system 10, the measurement system 20, the medical information system 30, and others via the network. The control unit 43 described later transmits and receives information to and from these devices via the communication unit 41.
The storage unit 42 is implemented by, for example, a semiconductor memory element such as a random access memory (RAM) or a flash memory or a storage device such as a hard disk or an optical disk. The storage unit 42 stores a learned model 55 generated by the control unit 43. The learned model 55 will be described later.
The control unit 43 may be implemented by, for example, a central processing unit (CPU) or a micro processing unit (MPU) executing a program stored inside the derivation device 40 (example of a diagnosis assist program) using a random access memory (RAM) or the like as a work area. Note that the control unit 43 may be implemented by, for example, an integrated circuit such as an application specific integrated circuit (ASIC) or a field programmable gate array (FPGA).
As illustrated in FIG. 2, the control unit 43 includes a measurement information acquiring unit 51, a diagnostic information acquiring unit 52, and a learning unit 53 and implements or executes a function or an action of information processing described below. Note that the internal configuration of the control unit 43 is not limited to the configuration illustrated in FIG. 2 and may be another configuration as long as the information processing described below is performed.
The measurement information acquiring unit 51 acquires measurement information, which is one piece of training data used for training of the learning model performed by the learning unit 53, from the server 12 and/or 22 via the communication unit 41, for example. The acquired measurement information may be accumulated in the storage unit 42 or the like as appropriate.
The diagnostic information acquiring unit 52 acquires diagnostic information, which is one piece of training data used for training of the learning model performed by the learning unit 53, from the server 12 and/or 22 via the communication unit 41, for example. The acquired diagnostic information may be accumulated in the storage unit 42 or the like as appropriate.

The learning unit 53 generates training data for training the learning model from the correspondence relationship between the measurement information acquired by the measurement information acquiring unit 51 and the diagnostic information acquired by the diagnostic information acquiring unit 52 and trains the learning model using the generated training data. As a result, the learned model 55 that has been trained is stored in the storage unit 42, for example, and is read as appropriate depending on the need.

Note that the training method of the learning model by the learning unit 53 may be based on any algorithm. For example, the learning unit 53 can generate the learned model 55 using various learning algorithms such as deep learning which is a machine learning method by a deep neural network, support vector machine, clustering, or reinforcement learning.

Furthermore, when estimation of a diagnosis result for a specific patient is requested by a user via the server 12/22, the learning unit 53 acquires at least one of measurement information or diagnostic information of the specified patient as input data and inputs the acquired data to the learned model 55, thereby causing the learned model 55 to derive an estimation result of diagnosis such as efficacy of a medicine or the health condition. The estimation result derived as a result is transmitted to the server 12/22, for example, and is displayed on the display device 14/24 under the control of the display control device 13/23.

1.3 Control Unit

Next, the control unit 43 in the derivation device 40 according to the present embodiment will be described in more detail.

As described above, the diagnosis assist system 1 is expected to support multimodal learning capable of integrating and learning data in which data formats acquired by various information acquiring devices are not unified in order to estimate, by machine learning, a diagnosis result of a disease such as "cancer" (such as the health condition of a patient or a subject, a medicine effective for treatment or effectiveness of the medicine (also referred to as efficacy)) that is caused by a very wide variety of factors complicatedly intertwined. The modal information mentioned here may be measurement information obtained from measuring devices 11, diagnostic information obtained from the medical information system 30, or others.

Therefore, in the present embodiment, it is made possible to perform multimodal learning capable of estimating a diagnosis result by integrating modal information (measurement information or diagnostic information) in which data formats (hereinafter, also simply referred to as formats) obtained from one or more measuring devices 11, the medical information system 30, and others are not unified. Furthermore, in the present embodiment, it is also made possible to present to a user, by multimodal learning, what type of measuring device 11 is more suitable for acquiring data in specifying a medicine effective for treatment and the efficacy of the medicine.

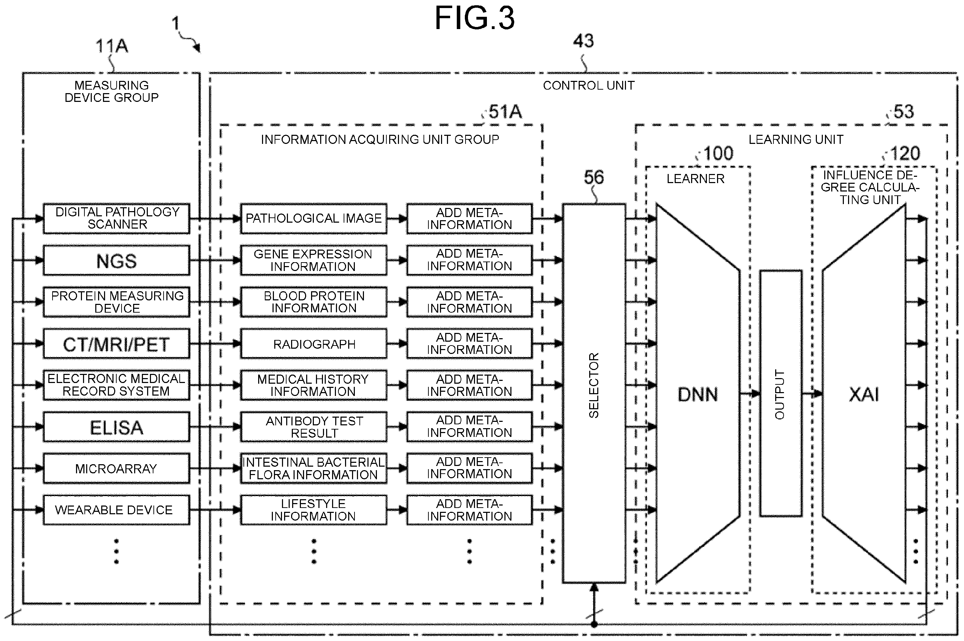

FIG. 3 is a block diagram illustrating a more detailed configuration example of the control unit according to the embodiment. However, in FIG. 3, the one or more measuring devices 11 in the measurement system 10/20 are illustrated as a measuring device group 11A. The measuring device group 11A may also include a medical information system 30 which is an electronic medical record system.

As illustrated in FIG. 3, the control unit 43 includes an information acquiring unit group 51A, a learning unit 53, and a selector 56.

The information acquiring unit group 51A includes, for example, the measurement information acquiring unit 51 and the diagnostic information acquiring unit 52 illustrated in FIG. 2 and acquires training data or modal information (measurement information and/or diagnostic information, also referred to as input data) used for estimation of a diagnostic result from the measuring devices 11 of the measuring device group 11A and/or the medical information system 30. For example, a pathological image is acquired from a digital pathology scanner, gene expression information from NGS, blood protein information from a protein measuring device, a radiograph from CT/MRI/PET, diagnostic information such as medical history information from the electronic medical record system (medical information system 30), an antibody test result from ELISA, intestinal bacterial flora information from a microarray, and lifestyle information is acquired from a wearable device. Note that the wearable device can include various devices capable of acquiring information regarding the daily lifestyle (hours of sleep, heart rate, number of steps, blood oxygen concentration, blood glucose level, or others) of a user, such as a smartphone or a smart watch.

Furthermore, the information acquiring unit group 51A may add meta-information to the acquired modal information. The meta-information may include, for example, information for identifying a patient corresponding to each piece of modal information, patient's disease information, test information or image information used for diagnosis, a diagnosis result, prescribed medicine, or others. Furthermore, in a case where the modal information is image data such as a pathological image, the meta-information may include information such as an annotation added to a specific region in the image data by a doctor, a pathologist, or the like. The meta-information may also include information generated from the modal information by image recognition or the like. However, it is not essential to add meta-information to the modal information, and the meta-information may be omitted. Furthermore, in a case where meta-information is added to the modal information, "modal information" in the following description may include "meta-information".

The selector 56 selects modal information to be used in multimodal learning of a learner 100 (learning model) or estimation of a diagnosis result using the learned learner 100 (learned model 55) from among a plurality of pieces of modal information that has been input on the basis of control information input from an influence degree calculating unit 120 to be described later. The selected modal information is input to the learning unit 53.

The learning unit 53 includes a learner 100 and an influence degree calculating unit 120.

The learner 100 is a learning model before learning or in the process of learning (may be the learned model 55 at the time of relearning or estimation of a diagnosis result) and may, for example, use a neural network such as a deep neural network (DNN).

The influence degree calculating unit 120 is a calculation unit that calculates the degree of influence given by each piece of modal information used by the learner 100 to derive (estimate the diagnosis result) the output (in the present description, this is a diagnostic result, which is for example a medicine effective for treatment or the efficacy of the medicine) in this derivation (estimation of the diagnosis result) and, for example, calculates the degree of influence of each piece of modal information using the explainable AI (XAI) technology. The calculated degree of influence is input to the selector 56 as control information, for example, and is used for selection of modal information to be used as training data and selection of modal information to be used for estimation of the diagnosis result. Furthermore, the calculated degree of influence may be input to the server 12/22 in the measurement system 10/20, for example, and may be used as information for presenting to a user what type of measuring device 11 is more suitable for acquiring data.

1.4 Neural Network Architecture for Multimodal Learning

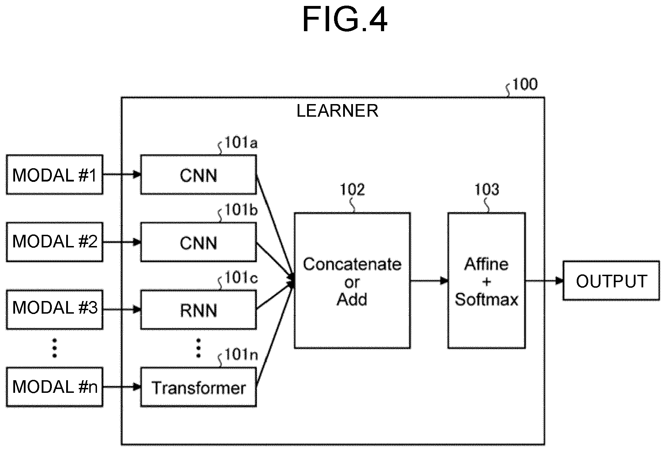

Next, a neural network architecture for multimodal learning incorporated in the learner 100 according to the present embodiment will be described. FIG. 4 is a diagram illustrating an example of a neural network architecture for multimodal learning incorporated in the learner according to the present embodiment. As illustrated in FIG. 4, according to the neural network architecture for multimodal learning according to the embodiment, the learner 100 includes a plurality of neural network units 101a to 101n arranged in parallel, a concatenation or addition unit 102, and an affine and softmax unit (also referred to as a derivation unit) 103.

Each of the neural network units 101a to 101n (hereinafter, in a case where the individual neural network units 101a to 101n are not distinguished, their symbols are denotes as '101') is a neural network that extracts features for deriving an estimation result (medicine or efficacy of a medicine in this example) on the basis of the modal information #1 to #n input to the respective neural network units and may be, for example, various neural networks corresponding to an information acquiring device that has acquired the modal information that is input to the neural network unit, such as a convolutional neural network (CNN), a recurrent neural network (RNN), or a transformer.

The concatenation or addition unit 102 is a coupling layer that concatenates or adds the features of the respective pieces of modal information #1 to #n output from the respective neural network units 101. For example, the concatenation or addition unit 102 converts each piece of the modal information #1 to #n output from each of the respective neural network units 101 into a one-dimensional vector and then concatenates and fully combines the pieces of modal information. Alternatively, the concatenation or addition unit 102 performs addition after matching the dimensions of the modal information #1 to #n output from the respective neural network units 101.

The affine and softmax unit 103 is an output layer that calculates and outputs a final estimation result (output) by executing an affine transformation and Softmax on the output from the concatenation or addition unit 102.

1.5 Method for Calculating Degree of Influence (Contribution Ratio) of Each Piece of Modal Information on Output

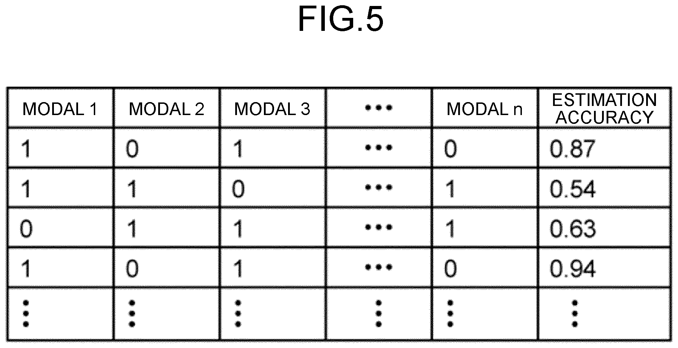

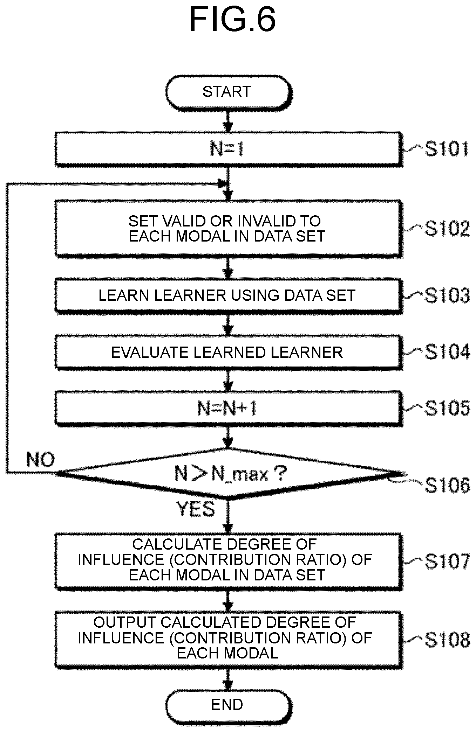

Next, a calculation method in which the influence degree calculating unit 120 calculates the degree of influence (contribution ratio) given by each piece of modal information in the derivation of the output (estimation of a diagnosis result) by the learner 100 will be described. FIG. 5 is a diagram illustrating an exemplary table used in calculation of the degree of influence according to the embodiment. FIG. 6 is a flowchart illustrating an example of an influence degree calculating operation according to the embodiment.

1.5.1 Management Table

As illustrated in FIG. 5, in the calculation of the degree of influence of each piece of modal information, for example, a management table is created which manages records for setting validity and invalidity of each piece of modal information in a data set and the accuracy (estimation accuracy) of output (estimation result) output from the learner 100 in a case where and a combination of valid modal information in each record is input. In the management table illustrated in FIG. 5, for example, validity and invalidity of each piece of modal information is managed by '1' and '0', respectively. Note that the management table may be created and managed by, for example, the learning unit 53 (for example, the influence degree calculating unit 120 and/or the selector 56, hereinafter, also referred to as a selection unit).

Each record of the management table also stores accuracy (estimation accuracy) of output (estimation result) in a case where one or more pieces of modal information set to valid in the record is input.

In the calculation of the degree of influence (contribution ratio) of each piece of modal information, a weight w for each piece of modal information for calculating the accuracy of output (estimation result) is calculated by executing linear regression with a regularization term such as ridge regression or Lasso regression on the basis of a combination of valid and invalid of modal information in each record of the created management table and the estimation accuracy of each record.

In this example, in the present embodiment, it can be determined that modal information having a weight w of a larger value has a positive influence on the output accuracy. On the other hand, it can be determined that modal information having a weight w of a small value has a low contribution ratio to the output accuracy, and modal information having a weight w of a negative value of has a negative influence on the output accuracy. Therefore, in the embodiment, a calculated weight w of each piece of modal information is output as the degree of influence (contribution ratio) on the output by the piece of modal information.

1.5.2 Flowchart

Next, the operation of the influence degree calculating unit 120 for calculating the degree of influence will be described using the above-described management table. As illustrated in FIG. 6, in the calculation of the degree of influence, the influence degree calculating unit 120 first sets a count value N of a counter (not illustrated) to 1 (step S101).

Next, the influence degree calculating unit 120 adds a record to the management table illustrated in FIG. 5 and stores '1' or '0' indicating validity or invalidity in a field corresponding to each piece of modal information in the added record (step S102). As the valid and invalid values set for each piece of modal information, for example, various values may be used such as values randomly generated using a pseudo-random number generator or the like (a sequence of '0' and '1' corresponding to the number of pieces of modal information) or a sequence selected in a predetermined order from a plurality of values generated in advance.

Next, the influence degree calculating unit 120 learns (trains) the learner 100 by using a data set including modal information for which validity is set in the record added in step S102 as input (step S103). Subsequently, the influence degree calculating unit 120 evaluates the learner 100 by calculating the estimation accuracy of the learned learner 100 on the basis of the estimation result output from the learner 100 in the learning in step S103 (step S104). The estimation accuracy calculated as a result of this is stored in the field of estimation accuracy in the record added in step S102.

Next, the influence degree calculating unit 120 increments the count value N by one (step S105) and determines whether or not the incremented count value N exceeds a preset upper limit value N_max (step S106). If the count value N does not exceed the upper limit value N_max (NO in step S106), the influence degree calculating unit 120 returns to step S102 and repeats the subsequent operations. On the other hand, if the count value N exceeds the upper limit value N_max (YES in step S106), the influence degree calculating unit 120 proceeds to step S107.

In step S107, the influence degree calculating unit 120 calculates the degree of influence (contribution ratio) of each piece of modal information in the data set on the basis of the created management table (step S107). Specifically, for example, as described above, the influence degree calculating unit 120 calculates a weight w for each piece of modal information for calculating the accuracy of output (estimation result) by executing linear regression with a regularization term such as ridge regression or Lasso regression on the basis of a combination of valid and invalid of modal information in each record of the created management table and the estimation accuracy of each record.

Then, the influence degree calculating unit 120 outputs the weight w of each piece of modal information calculated in step S107 as the degree of influence (contribution ratio) on the output by the piece of modal information (step S108) and ends this operation.

1.5.3 Another Example of Influence Degree (Contribution Ratio) Calculating Method

For calculation of the degree of influence of each piece of modal information regarding the output, for example, it is also possible to use a method using the SGDinfluence technology disclosed in Non-Patent Literature (Satoshi Hara, Atsushi Nitanda, and Takanori Maehara. "Data Cleaning for Models Trained with SGD." arXiv preprint arXiv: 1906.08473 (2019), NIPS2019).

Alternatively, for example, a method using an influence function can also be used for calculation of the degree of influence. The influence function is obtained by formulating the influence of each piece of data (modal information in the present description) in a data set on a model (parameter) (see, for example, Non-Patent Literature (Pang Wei Koh and Percy Liang. "Understanding Black-box Predictions via Influence Functions." Stanford University, ICML2017)). Hereinafter, measurement of the degree of influence using the influence function will be described. Note that the influence function can also be used as a method for describing a black box model of machine learning, for example.

In the description, as an example, a case is examined in which an estimation problem in which input x is an image and output y is a label is solved by machine learning. It is based on the premise that each image is associated with a correct answer label. For example, presuming that there are n sets (data sets) of an image and a label (n is any natural number), each labeled image z (which may be simply described as "image z") can be expressed by the following Equation (1).

$$z_1, z_2, \cdots, z_n \qquad z_i = (x_i, y_i) \in X \times Y \qquad (1)$$

Here, let a loss at a parameter $\theta \in \Theta$ of the model at a certain point z (image z) be L(z, $\theta$), and an empirical risk in all n pieces of set data is expressed by the following Expression (2).

$$\frac{1}{n} \sum_{i=1}^{n} L(z_i, \theta) \qquad (2)$$

The empirical risk minimization expressed by Expression (2) means to find (determine) a parameter that minimizes the loss. Therefore, the parameter for minimizing the empirical risk expressed by Expression (2) can be derived from the following Equation (3).

$$\hat{\theta} \equiv \arg\min_{\theta \in \Theta} \frac{1}{n} \sum_{i=1}^{n} L(z_i, \theta) \qquad (3)$$

Therefore, the influence degree calculating unit 120 calculates the parameter that minimizes the empirical risk by calculating the left side of Equation (3).

Note that a parameter (variable) in which "^" is added above a certain character, such as the parameter (variable) in which "^" (hat) is added above "θ" in the left side of Equation (3), indicates, for example, an estimation value. Hereinafter, when referring to the parameter (variable) in which "^" is added to "θ" in the left side of Equation (3) in a sentence, it is denoted as "θ^" in which "^" follows "θ".

Next, how the calculation is performed in the influence degree calculating unit 120 will be described with the aim of understanding the degree of influence of data that is a training point of the learning model on the premise that the empirical risk can be second-order differentiated and is a convex function with respect to the parameter θ. In this description, first, in a case where there is no data of a certain training point, what type of influence can be given to the learning model is examined.

In a case where a certain training point z (image z) is removed from the learning model, it can be expressed as the following Equation (4).

$$\hat{\theta}_{-z} \equiv \arg\min_{\theta \in \Theta} \frac{1}{n} \sum_{z_i \neq z} L(z_i, \theta) \qquad (4)$$

For example, the influence degree calculating unit 120 calculates a parameter (the left side of Equation (4)) in a case where learning is performed using Equation (4) without using the certain learning data (image z). For example, the degree of influence is a difference between a case where the training point z (image z) is removed and a case where there are all data points including the training point z. This difference is expressed by the following Expression (5).

$$\hat{\theta}_{-z} - \hat{\theta} \qquad (5)$$

Here, if recalculation is performed for the case where the image z is removed, the calculation cost is quite high. Therefore, using the influence function, the influence degree calculating unit 120 performs calculation, without recalculating (relearning) the case where the image z is removed, by effective approximation as described below.

This idea is a method of calculating a change in the parameter based on the premise that the image z is weighted by $\varepsilon$ that is minute. Here, a new parameter (the left side of Equation (6)) is defined using the following Equation (6).

$$\hat{\theta}_{\varepsilon,z} \equiv \arg\min_{\theta \in \Theta} \frac{1}{n} \sum_{z_i \neq z} L(z_i, \theta) + \varepsilon L(z, \theta) \qquad (6)$$

By utilizing the results of a prior study by Cook and Weisberg in 1982, the degree of influence of the image z weighted with the parameter θ^ ((left side of Equation (3))) can be expressed as the following Equations (7) and (8).

$$I_{up,params}(z) \equiv \frac{d\hat{\theta}_{\varepsilon,z}}{d\varepsilon}\Big|_{\varepsilon=0} = -H_{\hat{\theta}}^{-1}\nabla_\theta L(z, \hat{\theta}) \qquad (7)$$

$$H_{\hat{\theta}} \equiv \frac{1}{n}\sum_{i=1}^{n}\nabla_\theta^2 L(z_i, \hat{\theta}) \qquad (8)$$

Note that the prior study by Cook and Weisberg are disclosed in, for example, Non-Patent Literature (Cook, R. Dennis and Sanford Weisberg. "Residuals and Influence in Regression." New York: Chapman and Hall. 1982).
In the above, Equation (7) represents an influence function corresponding to the certain image z. For example, Equation (7) represents a change amount of a parameter with respect to the minute ε. In addition, Equation (8) represents Hessian (Hessian matrix). Here, it is presumed that the Hessian matrix has a positive definite, and there is also an inverse matrix. Presuming that removing the data point z (image z), which is a certain point, is the same as being weighted by "ε = -1/n", a change in the parameter when the image z is removed can be approximately expressed by the following Expression (9).

$$\hat{\theta}_{-z} - \hat{\theta} \approx -\frac{1}{n}I_{up,params}(z) \qquad (9)$$

Therefore, by calculating Expression (9), the influence degree calculating unit 120 can measure (obtain) the degree of influence of the case where the data point z (image z) is removed without performing relearning.
Then, the influence degree calculating unit 120 measures (obtains) the degree of influence on the loss at the certain test point $z_{test}$ using the following Equation (10-1) to (10-3).

$$I_{up,loss}(z, z_{test}) \equiv \frac{dL(z_{test}, \hat{\theta}_{\varepsilon,z})}{d\varepsilon}\Big|_{\varepsilon=0} \qquad (10-1)$$

$$\equiv \nabla_\theta L(z_{test}, \hat{\theta})^T \frac{d\hat{\theta}_{\varepsilon,z}}{d\varepsilon}\Big|_{\varepsilon=0} \qquad (10-2)$$

$$\equiv -\nabla_\theta L(z_{test}, \hat{\theta})^T H_{\hat{\theta}}^{-1}\nabla_\theta L(z, \hat{\theta}) \qquad (10-3)$$

In this manner, the degree of influence of the weighted image z at the certain test point $z_{test}$ can be formulated. Therefore, the influence degree calculating unit 120 can measure (obtain) the degree of influence of each piece of data (modal information) in the learning model by this calculation. For example, since the right side of Equation (10-3) includes a gradient with respect to the loss of certain data, an inverse matrix of the Hessian, a gradient of the loss of certain learning data, and others, for example, the influence of certain data (modal information) on the estimation (loss) of the model can be obtained by Equation (10-3). Note that the above is an example, and the influence degree calculating unit 120 may execute various calculations as appropriate and measure the degree of influence of each piece of modal information regarding the learning.
Furthermore, in this example, the example where the degree of influence on the output of each modal is calculated as a continuous value has been described, however, without being limited thereto, for example, the influence degree calculating unit 120 may calculate the degree of influence of each piece of modal information regarding the output in two stages of "with influence/no influence", may calculate in three stages of "with favorable influence/no influence/with adverse influence", or may calculate with discrete values of four or more stages.

1.6 About Deletion And Addition of Modal Information

The degree of influence of each piece of modal information calculated as the above can be used as, for example, modal information used for learning (training) of the learner 100 or estimation of a diagnosis result, that is, information for selecting modal information to be validated from among modal information included in the data set. Note that deletion or addition (corresponding to being valid or invalid) of the modal information input to the learner 100 is implemented by, for example, the selector 56 illustrated in FIG. 3. The deletion or addition of modal information may be manually set to the selector 56 by a user from, for example, the server 12/22 of the measurement system 10/20 or the like, may be automatically set by the selector 56 on the basis of the degree of influence from the influence degree calculating unit 120, or may be set by the influence degree calculating unit 120 to the selector 56 on the basis of the degree of influence.

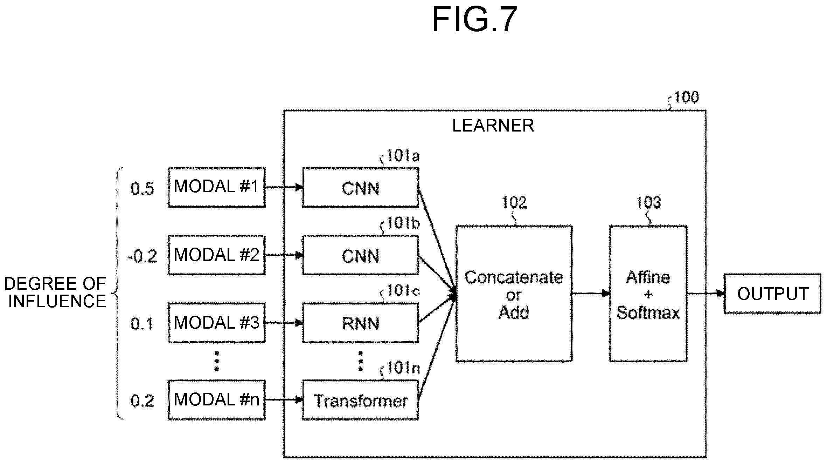

FIG. 7 is a diagram for explaining deletion and addition of modal information according to the embodiment. As illustrated in FIG. 7, for example, the influence degree calculating unit 120 calculates the degree of influence having a larger absolute value for modal information having a larger influence on the output estimation accuracy, calculates a positive degree of influence for modal information having a favorable influence, and calculates a negative degree of influence for modal information having an adverse influence.

Therefore, in a case where the influence degree calculating unit 120 or the selector 56 automatically sets deletion or addition of modal information, the influence degree calculating unit 120/the selector 56 may set a predetermined number of pieces of modal information, set in advance in descending order of values on the basis of the degree of influence of each piece of modal information, to be valid (for example, add to a data set) and set the other pieces of modal information to be invalid (for example, delete from the data set) or may set modal information, of which degree of influence is equal to or greater than a preset threshold value, to be valid (for example, add to data set) and set modal information of which degree of influence is less than the threshold value to be invalid (for example, delete from the data set). Note that a user may be able to set, as desired, validity or invalidity of the modal information of which degree of influence is less than the threshold value. Alternatively, for example, on the basis of the degree of influence of each piece of modal information, the influence degree calculating unit 120/the selector 56 may invalidate modal information included in a preset ratio (for example, 1%, 3%, 10%, etc.) in an ascending order (namely, from the side with an adverse influence on the output) or may validate modal information included in a preset ratio (for example, 1%, 3%, 100, etc.) in a descending order (namely, from the side with a favorable influence on the output). However, the present invention is not limited to these rules, and deletion or addition of modal information may be executed on the basis of various rules.

Note that input to the learner 100 may be omitted for invalidated modal information as described above, or dummy data may be input instead of the modal information. The dummy data that is input as a substitute may be data that does not affect the output (the degree of influence is zero) or data that has a sufficiently small influence on the output. At this point, a data format of the dummy data may be the same as the format of the alternative modal information or may be a different format.

Furthermore, the data set from or to which the modal information has been deleted or added on the basis of the degree of influence may be used for relearning of the learner 100. Furthermore, the accuracy of the learner 100 may be enhanced by repeating, from the calculation of the degree of influence, deletion or addition of modal information and relearning of the learner 100. Note that the number of times of repetition of relearning may be the number of times with which a sufficient accuracy of the learner 100 is expected to obtain. At this point, the learner 100 may be trained in a plurality of patterns by gradually increasing or decreasing the number of pieces of or the ratio of modal information to be validated or invalidated, and a learner having the highest estimation accuracy may be selected from a plurality of learners 100 generated by the training.

Furthermore, in the relearning of the learner 100, for example, a record in which validity or invalidity of each piece of modal information is set on the basis of the degree of influence is added to the management table illustrated as an example in FIG. 5. Alternatively, a management table including a record in which validity or invalidity of each piece of modal information is set on the basis of the degree of influence is newly generated. The learning unit 53 improves the accuracy of the learner 100 by relearning the learner 100 on the basis of the new record.

At this point, the learning unit 53 may generate, on the basis of the record that has been added on the basis of the degree of influence, one or more new records similar to the setting content of validity or invalidity of this record, add the new record(s) to the management table, and relearn the learner 100 on the basis of each of the one or more added records. This makes it possible to calculate the degree of influence more accurately. Note that "similar to the setting content" may be that, between the record that has been added on the basis of the degree of influence and the newly added record(s) (or between the newly added record(s) and another newly added record), modal information for which validity is set and modal for which invalidity is set do not completely match, but the number of pieces of the inconsistent modal information is small (for example, one to three).

1.7 Visualization of Degree of Influence of Each Piece of Modal Information

Next, a case where the degree of influence of each piece of modal information calculated as described above is visualized and presented to a user will be described. For example, by presenting the calculated degree of influence of each piece of modal information to the user, it is made possible to indicate, to the user, information regarding which measuring device 11 is useful to perform measurement and which measuring device 11 is useless or has an adverse influence to perform measurement and information regarding which modal information is preferable to be validated among modal information included in a data set that has been already acquired. Note that visualization of the degree of influence may be executed, for example, in the derivation device 40 or may be executed in the server 12/22 or the display control device 13/23 in the measurement system 10/20. In other words, the derivation device 40, the server 12/22 in the measurement system 10/20, or the display control device 13/23 may constitute an influence degree presenting unit that visualizes and displays the degree of influence of each piece of modal information to the user. Furthermore, the visualized degree of influence may be displayed on, for example, the display device 14/24 in the measurement system 10/20 or may be displayed on another display device.

(Visualization of Degree of Influence of Each Piece of Modal Information)

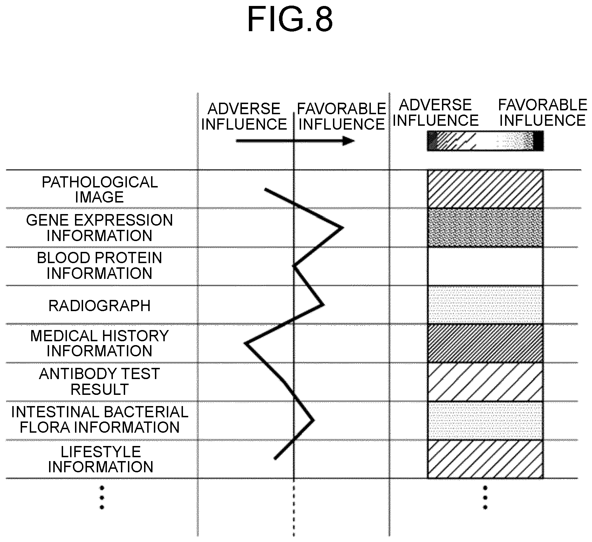

FIG. 8 is a diagram illustrating an example of a concept visualizing the degree of influence for every piece of modal information according to the embodiment. The degree of influence for each piece of modal information exemplified in the left column of FIG. 8 may be presented to a user by a polygonal line as illustrated in the middle column of FIG. 8 or may be indicated by visual effects such as color or hatching as illustrated in the right column of FIG. 8, for example. Moreover, the degree of influence for each piece of modal information may be presented in various modes in which the user can recognize the degree of influence such as numerical values or characters (large, small, etc.).

(Visualization of Degree of Influence of Other Modal Information with Respect to Specific Modal Information)

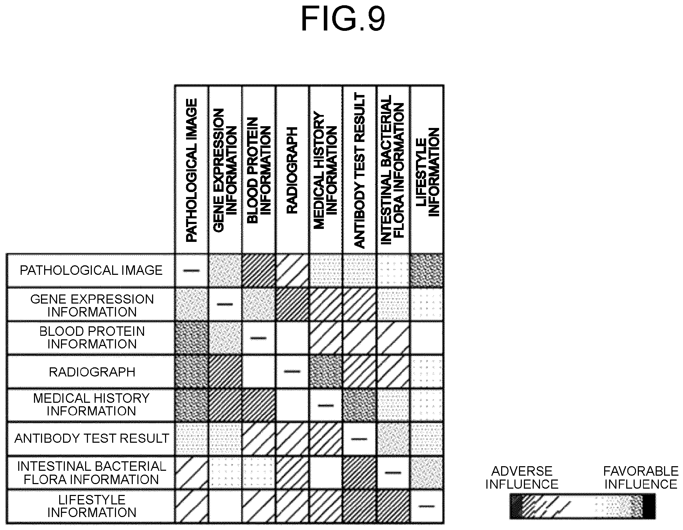

FIG. 9 is a diagram illustrating an example of a concept in which the degree of influence (namely, congeniality among pieces of modal information in inference using a learner) of other modal information with respect to specific modal information according to the embodiment is visualized. In FIG. 9, the degree of influence of each piece of modal information listed on the left side in the table is visualized with respect to each piece of modal information listed on the upper side in the table. Note that, in FIG. 9, a case where the degree of influence of other modal information with respect to each piece of modal information is visualized by color is illustrated, however, without being limited thereto, the degree of influence may be displayed in various modes that can be recognized by the user, such as numerical values or characters (large, small, etc. )

(Visualization of Affected Region in Modal Information)

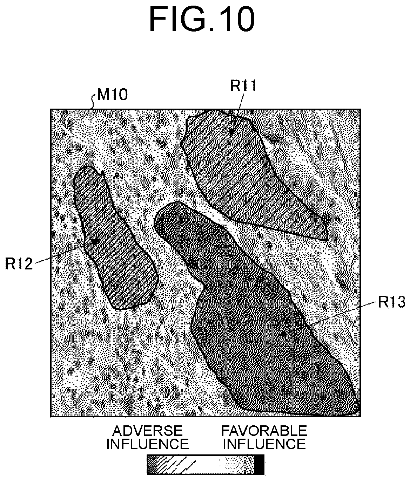

FIG. 10 is a diagram illustrating an example of a concept obtained by visualizing affected regions in modal information according to the embodiment. Note that FIG. 10 illustrates an example in which the modal information is a pathological image obtained by photographing a tissue piece collected from a patient. As illustrated in FIG. 10, in a case where the influence degree calculating unit 120 can calculate the degree of influence for each region in each piece of modal information, for at least one piece of modal information of modal information included in a data set, a region in the modal information that has affected the derivation of the output may be visualized and presented to the user by an effect such as color, hatching, or emphasizing the contour. In FIG. 10, in modal information M10 which is a pathological image, regions R11 and R12 having an adverse influence and a region R13 having a favorable influence are indicated by hatching that indicates the degree of influence. Note that, in a case where the modal information is character information such as an electronic medical record, the degree of influence may be visualized by color coding of characters or visual effects such as showing in bold or highlighting.

(Visualization of meta-information Affected in Modal Information)

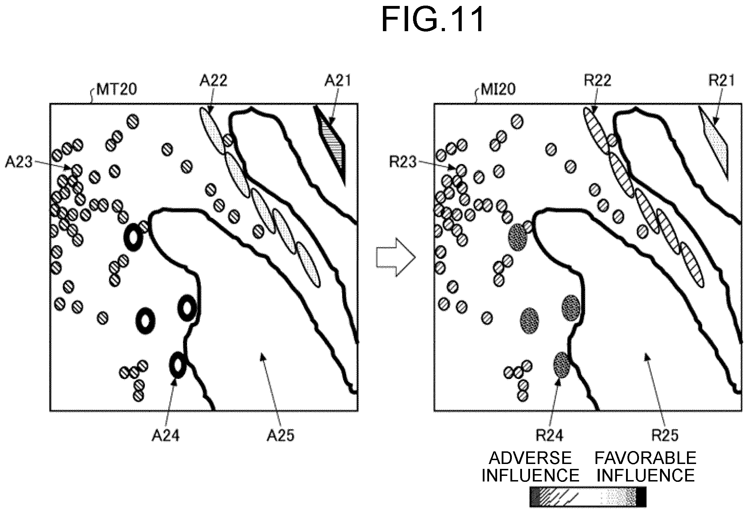

Furthermore, in a case where modal information includes meta-information, the meta-information affected in the modal information may be visualized and presented to the user. FIG. 11 is a diagram illustrating an example of a concept in which meta-information affected in modal information is visualized according to the embodiment. Note that FIG. 11 illustrates a case where the modal information is a pathological image obtained by photographing a tissue piece collected from a patient and the meta-information is annotation added to a specific region (for example, a region such as cells) in the pathological image. As illustrated in FIG. 11, in a case where the influence degree calculating unit 120 can calculate the degree of influence for each piece of meta-information in each piece of modal information, meta-information that has affected the derivation of output in the modal information may be visualized and presented to the user by effects such as color, hatching, and emphasizing the contour. In FIG. 11, adversely affecting annotation A21 and favorably affecting annotation A22 to A25 in metadata MT20 added to the pathological image are expressed as regions R21 to R25 visualized by hatching representing the degree of influence thereof in an image MI20 presented to the user. Note that, in FIG. 11, a symbol is assigned to a representative one of one or more regions to which the same type of annotation is assigned, and regions assigned with other annotation are also similarly denoted. Furthermore, in a case where the presentation mode of meta-information to the user is a character string or the like, the degree of influence thereof may be visualized by an effect such as color coding of characters, showing in bold, highlighting, or the like.

1.8 Visualization of Affected Region in Modal Information

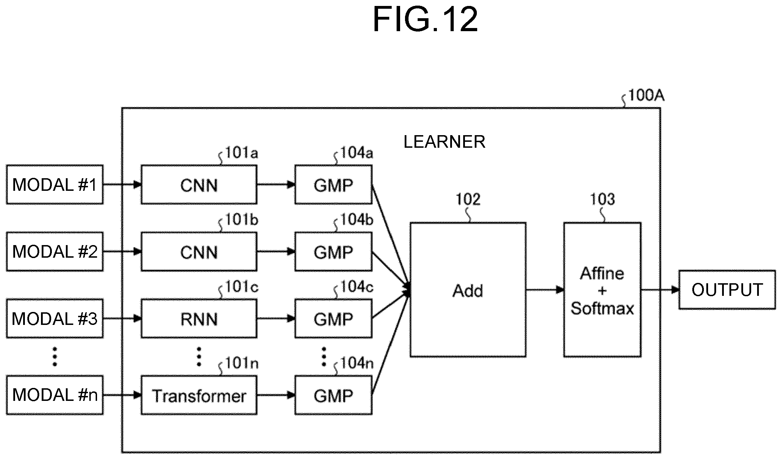

Next, visualization of an affected area in modal information will be described. For visualizing an affected area in modal information, for example, a method such as multiple-instance learning (MIL) can be used. FIG. 12 is a diagram illustrating an example of a neural network architecture for multimodal learning incorporated in a learner according to a modification of the present embodiment and is a diagram illustrating an example of a neural network architecture for multimodal learning that visualizes an affected region in modal information using the MIL.

As illustrated in FIG. 12, a learner 100A according to the present modification has a configuration in which GMP units 104a to 104n (hereinafter, their symbols are denoted as '104' when individual GMP units 104a to 104n are not distinguished) are added as subsequent stages of neural network units 101a to 101n in a neural network architecture for multimodal learning similar to that of the learner 100 illustrated as an example in FIG. 4.

Each of the GMP units 104 is a pooling layer that narrows down the number of neurons to the number of categories to be estimated by performing global max pooling on output from a neural network unit 101 in the preceding stage in the spatial-temporal direction. Specifically, for example, each of the GMP units 104 narrows down the number of neurons of the output to the number of categories to be estimated by using the maximum value in the spatial-temporal direction of output from one of the neural network units 101. As a result, the output from each of the GMP units 104 is narrowed down to an affected area in the modal information.

The concatenation or addition unit 102 adds the neurons of which number of dimensions is the number of categories output from the GMP units 104. Therefore, output D_add from the concatenation or addition unit 102 is neurons of which number is the number of categories to be estimated (for example, two of with efficacy and without efficacy). The affine and softmax unit 103 derives an estimation result (such as a medicine effective for treatment or the efficacy of the medicine) as the final output by executing Softmax on the output D_add from the concatenation or addition unit 102.

Note that an affected region in the modal information output from each of the GMP units 104 is visualized by a visual effect and presented to the user, for example. For example, in a case where the modal information is image data, it is made possible for the user to visually confirm which region in the image data has affected the output by displaying the image data and then giving visual effects such as color coding, emphasizing the contour, or enclosing with a rectangular bold line to a region in the image data that has affected the output. Furthermore, for example, in a case where the modal information is text data, it is made possible for the user to visually confirm which character or character string in the text data has affected the output by displaying the text data and then giving visual effects such as color coding, showing in bold, or highlighting the character or the character string that has affected the output in the text data.

Furthermore, in the present modification, the affected region in the modal information output from each of the GMP units 104 may be used for a function of automatically adding annotation to the modal information. That is, for example, the information acquiring unit group 51A (see FIG. 3) may automatically add annotation to a region that has affected the output in each piece of the modal information output from each of the GMP units of the learner 100A.

1.8.1 About Narrowing Down Affected Region in Modal Information

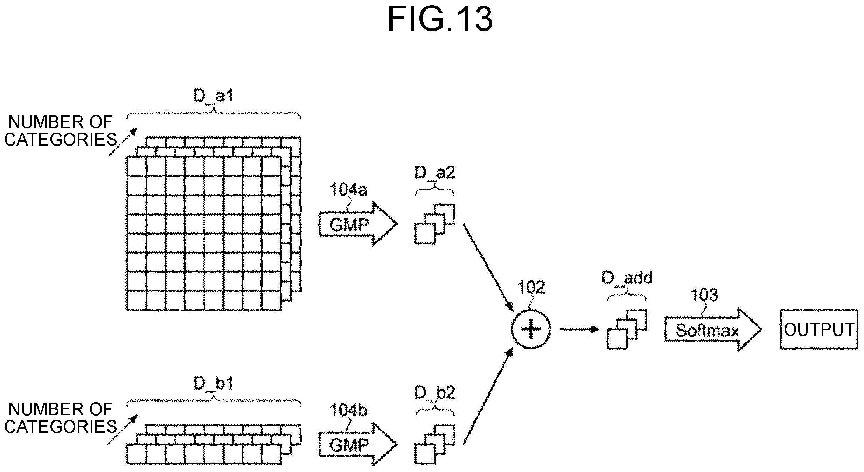

Here, a flow of narrowing down a region having affected the output from each piece of modal information will be described using a specific example. FIG. 13 is a diagram for explaining the specific example of a flow for narrowing down a region having affected output from each piece of modal information. In this example, each of the neural network units 101 outputs neurons of which number is spatial-temporal $\times$ the number of categories to be estimated (for example, two of with efficacy and without efficacy).

As illustrated in FIG. 13, in the specific example, it is based on the premise that the learner 100A includes two systems of neural networks, that one of the neural network units, which is a neural network unit 101a, handles image data having a two-dimensional (vertical $\times$ horizontal) spatial extent as modal information, and that the other neural network unit 101b handles text data having a one-dimensional (horizontal) spatial extent. In this case, output D_a1 of the neural network unit 101a is data having a structure of height $\times$ width $\times$ the number of categories, and output D_b1 of the neural network unit 101b is data having a structure of width × the number of categories.

For example, the GMP unit 104a uses the maximum value in the spatial-temporal direction with respect to the output D_a1 output from the neural network unit 101a. As a result, the number of neurons of output D_a2 from the GMP unit 104a is narrowed down to the number of categories to be estimated.

Similarly, the GMP unit 104b uses the maximum value in the spatial-temporal direction with respect to the output D_b1 output from the neural network unit 101b, for example. As a result, the number of neurons of output D_b2 from the GMP unit 104b is narrowed down to the number of categories to be estimated which is the same as the number of neurons of the output D_a2 from the GMP unit 104a.

1.9 Utilization of Wearable Devices

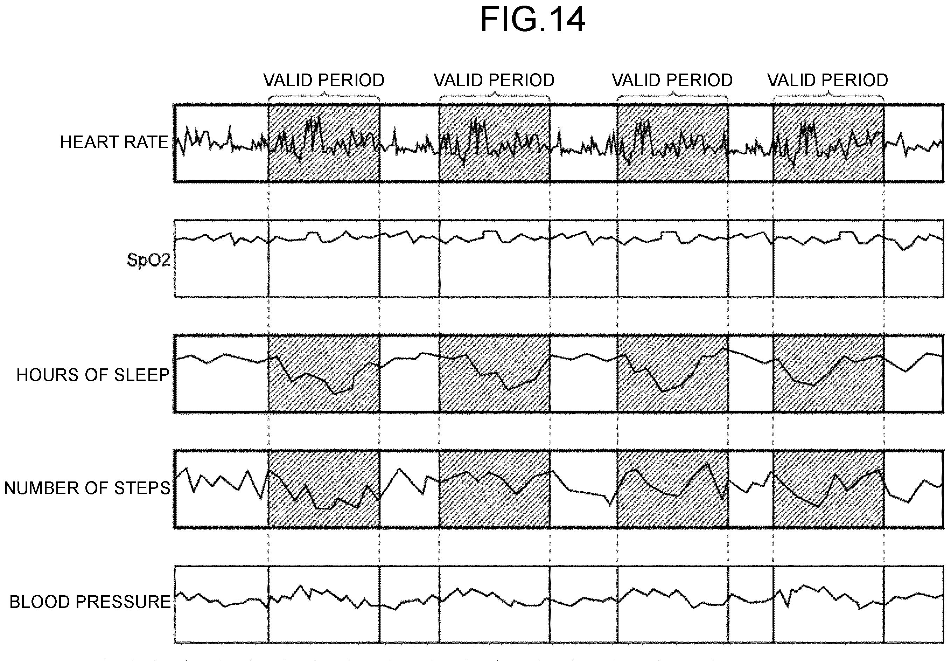

Next, a case where a wearable device, which is one of the measuring devices 11, is used for estimation of a diagnosis result will be described. FIG. 14 is a diagram illustrating an example of sensor data (modal information) acquired by a plurality of wearable devices.

As illustrated in FIG. 14, a wearable device such as a smartphone or a smartwatch is capable of acquiring information regarding a daily lifestyle (hours of sleep, heart rate, number of steps, blood oxygen concentration, blood glucose level, or others) of the user and is one of information acquiring devices effective in extracting and evaluating, for a certain period of time, information which fluctuates day to day and cannot be acquired by information acquiring devices that acquire information at a certain moment as a snapshot, such as a digital pathology scanner.

Sensor data acquired from such a wearable device alone has a low capability to estimate an optimal treatment method for a patient's condition with high accuracy. However, in a case where a plurality of pieces of sensor data such as a heart rate, hours of sleep, and the number of steps is combined, it can be expected that it becomes possible to estimate and determine a treatment method more accurately reflecting the patient's condition.

Therefore, as in the present embodiment, by performing multimodal learning by adding a plurality of wearable devices to the measuring devices 11, it becomes possible for the influence degree calculating unit 120 to specify which wearable device has a favorable influence on the accuracy of a medicinal efficacy estimation and to also specify which period (valid period) of the sensor data, as time-series information, has the most favorable influence. Note that, for specifying the valid period, for example, a method similar to the method of specifying an affected area in modal information described above may be used.

As described above, by performing multimodal learning by adding the plurality of wearable devices to the measuring devices 11, wearable devices required at the time of inference at the clinical site can be narrowed down, and in addition, by giving an instruction, to each of the wearable devices, as to which period of sensor data is to be extracted, it is made possible to estimate efficacy of a medicine in a realistic calculation time.

1.10 Specific Example of Flow from Multimodal Learning to Derivation of Estimation Result

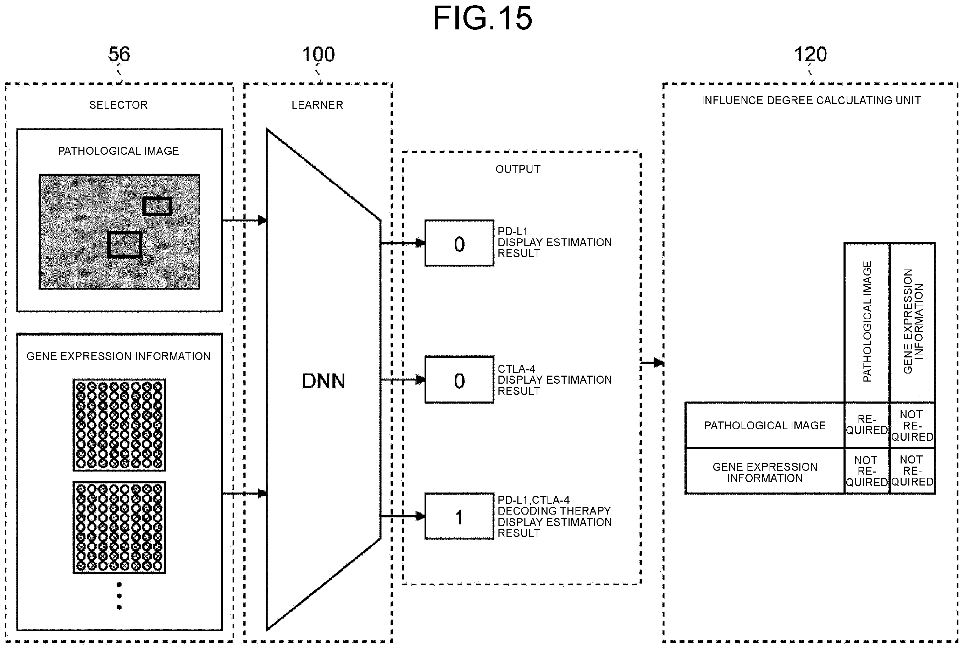

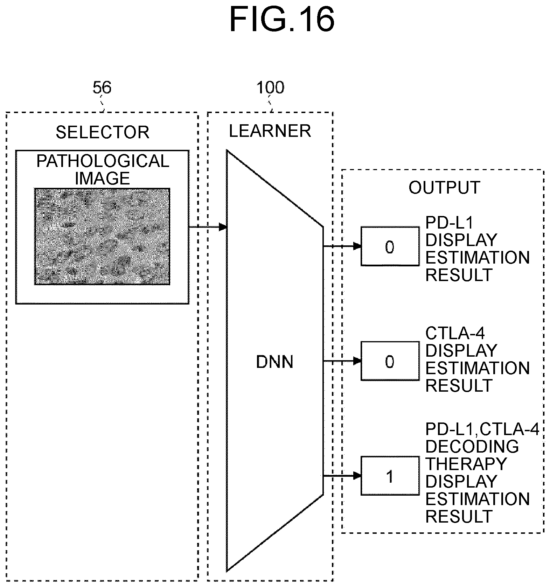

Next, a flow from multimodal learning to derivation of an estimation result according to the present embodiment will be described using a specific example. FIGS. 15 and 16 are diagrams for explaining a specific example of the flow from multimodal learning to derivation of an estimation result according to the embodiment. Note that, in FIGS. 15 and 16, configurations related to the present description are extracted and illustrated. Incidentally, FIG. 15 illustrates an example in which a pathology slide image (also simply referred to as a pathological image) and a combination of gene expression information and a medicinal efficacy label corresponding thereto are used as training data.

In the example illustrated in FIG. 15, at the time of learning of the learner 100, multimodal learning of images of individual cells in the pathological image and gene expression information corresponding thereto is performed together with a medicinal efficacy label. As a result, a small region in a cell nucleus is subjected to image analysis in combination with gene analysis at the single cell level, and thus it is possible to cause the learner 100 to learn to consider the correlation between an abnormality in gene expression and a shape change at the cell level.

Thereafter, when the learning of the correlation between the type of cellular atypia and the change in expression due to a specific gene mutation sufficiently progresses, it can be seen that the influence degree calculating unit 120 no longer needs to learn the pathological image and the gene expression information by multimodal information by being based on the degree of influence of each piece of modal information calculated each time of learning. For example, in the example illustrated in FIG. 15, it can be determined that the influence degree calculating unit 120 no longer needs the gene expression information for estimation of a diagnosis result from the calculated degree of influence of each piece of modal information.

In such a state, without using high-cost gene expression information at the clinical site, it is possible to specify a medicine optimal for treatment of "cancer" by performing inference only with the pathological image. Therefore, as illustrated in FIG. 16, at the time of deriving the estimation result, only the pathological image (image of individual cells) is input from the selector 56 to the learner 100.

1.11 Summary

As described above, according to the present embodiment, since the learner 100 performs multimodal learning by integrating modal information acquired by the plurality of measuring devices 11, it is possible to derive an estimation result of a diagnosis such as efficacy of a medicine or health condition by integrating data measured by the plurality of devices.

Furthermore, according to the present embodiment, it is possible to calculate the degree of influence of each piece of modal information and each region in each piece of modal information, and thus, it is possible to select modal information necessary for learning by the learner 100 or deriving an estimation result. This makes it possible to generate the learner 100 capable of deriving a more accurate estimation result. Furthermore, by presenting the degree of influence calculated by the influence degree calculating unit 120 to the user or presenting a measuring device 11 recommended on the basis of the calculated degree of influence to the user, it is made possible to reduce labor and cost at the time of measurement, and thus it is possible to reduce the burden on a doctor, a pathologist, a patient, or others. At this point, input data used for estimation of the diagnosis result may be modal information of the same type as that of the learning data used for training of the learner 100, may be modal information the number of types of which being smaller than the number of types of modal information included in the learning data, or may be modal information of a type different from that of the modal information included in the learning data.

Furthermore, according to the present embodiment, since it is possible to narrow down genes correlated with efficacy of a medicine from among a large number of genes on the basis of the calculated degree of influence of each piece of modal information, and thus, for example in order to obtain a biomarker having higher accuracy in medicinal efficacy estimation by adding information regarding the positional relationship of the expression site of a molecule synthesized from the gene, it is possible to recommend the user to add an immunostaining image of the molecule or to automatically select from an existing data set.

2. Second Embodiment

First, a second embodiment will be described in detail by referring to the drawings. In the first embodiment, the example where the learner 100 performs multimodal learning using modal information of various formats has been described. However, in a case where formats of pieces of modal information are different in multimodal learning, there is a possibility that the data set or the learner itself is redundant, whereby the learning cost increases. Therefore, in the present embodiment, it is made possible to suppress an increase in learning cost in a case where multimodal learning is performed using modal information of two or more types of formats.

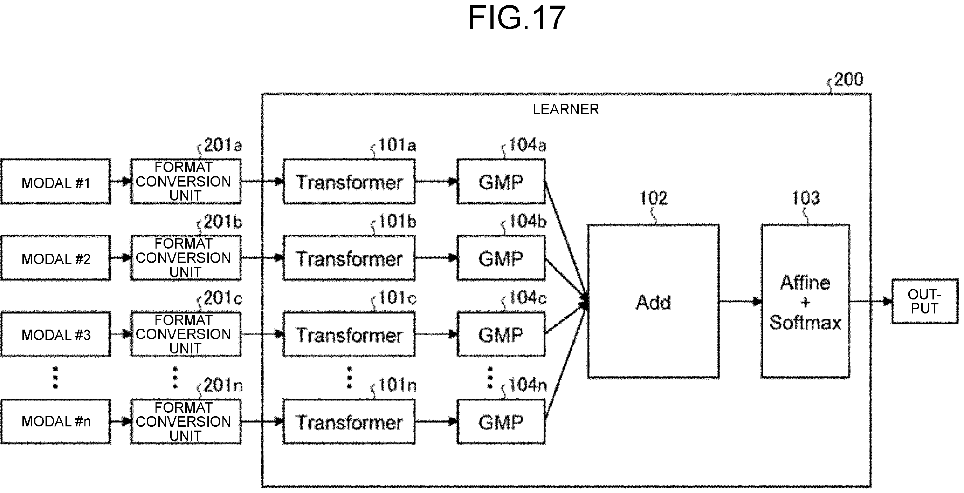

FIG. 17 is a diagram illustrating an example of a neural network architecture for multimodal learning incorporated in a learner according to the present embodiment. As illustrated in FIG. 17, in the neural network architecture for multimodal learning according to the embodiment, for example, in a configuration similar to that of the neural network architecture of the learner 100A described using FIG. 12 in the first embodiment, format conversion units 201a to 201n (hereinafter, in a case where the individual format conversion units 201a to 201n are not distinguished, their symbols are denoted as '201') are arranged in input stages of modal information #1 to #n to respective neural network units 101a to 101n. Furthermore, the neural network units 101a to 101n are configured by the same neural network (in FIG. 17, transformer) in order to use modal information of a unified format.

Note that the neural network architecture for multimodal learning of a learner 200 according to the embodiment is not limited to the neural network architecture for multimodal learning of the learner 100A exemplified in FIG. 12 and may be various modified such as the neural network architecture for multimodal learning of the learner 100 illustrated in FIG. 4. Furthermore, the format conversion units 201 may be, for example, included in the information acquiring unit group 51A or included in the learner 200.

Each of the format conversion units 201 converts the format of modal information input thereto into a predetermined common format. For example, in a case where the common format is image data of a fixed size, the format conversion units 201 to which text data is input as modal information convert the text data into image data of the fixed size. Alternatively, the format conversion units 201 to which waveform data such as the heart rate is input convert the waveform data into image data by generating a graph from the waveform data, for example.

Note that, in a case where text data is converted into image data, for example, a conversion table may be prepared in advance which associates a specific word, phrase, or the like (hereinafter, simply referred to as a keyword) that can be included in the text data with a specific pixel address in the image data, and the image data may be generated from the text data using the conversion table. In this case, the text data may be converted into image data by storing, for example, a bit value "255" in a pixel corresponding to a keyword included in the text data and storing, for example, a bit value "0" in other pixels.

As described above, by unifying the format of the modal information input to the neural network units 101, it is possible to suppress redundancy of the data set and the learner itself, thereby making it possible to suppress an increase in the learning cost.

Note that, in the present embodiment, the case where the formats of all the modal information input to the learner 200 are unified has been exemplified, however, it is not limited thereto, and it is sufficient that the number of types of formats of the modal information that is input is reduced by at least one. For example, the format conversion unit 201 may reduce the number of types of input formats by converting waveform data such as hours of sleep, the heart rate, blood oxygen concentration, or blood glucose level into unified waveform data. In addition, the format conversion unit 201 may reduce the number of types of input formats by converting different types of image data such as a pathological image and a radiograph, image data having different sizes, resolutions, number of colors, number of tones, or others acquired by digital pathology scanners of different models and the like into image data of a fixed size. Further, the format conversion unit 201 may reduce the number of types of input formats by translating text data such as medical history information created in different languages such as Japanese and English into a common language (for example, English).

3. Third Embodiment

Next, a workflow in a case where the diagnosis assist system 1 according to the above embodiment is actually introduced into medical setting will be described with an example.

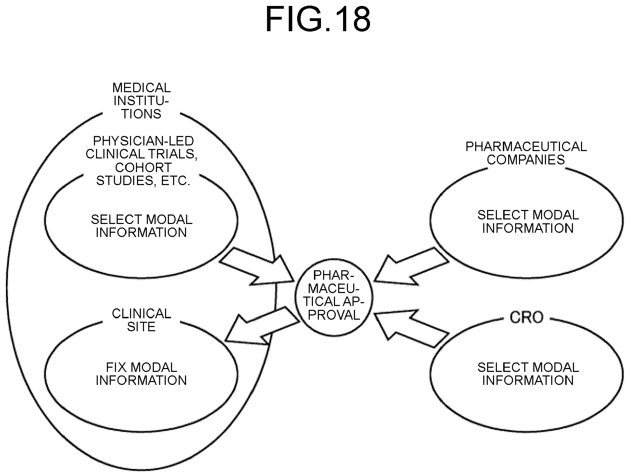

FIG. 18 is a schematic diagram illustrating an example of medical setting into which the diagnosis assist system 1 according to the embodiment is introduced. The example illustrated in FIG. 18 illustrates a case where each of research departments or the like of medical institutions, pharmaceutical companies, and contract research organizations (CROs) independently train the learner 100 using multimodal information obtained in physician-led clinical trials, cohort studies, marker searches, clinical trials, or the like, and one or more of the learners 100 trained independently are introduced as medical devices into a clinical site in a medical institution after obtaining a pharmaceutical approval based on a predetermined law of each country (hereinafter, simply referred to as a pharmaceutical approval) such as Act on Securing Quality, Efficacy and Safety of Products Including Pharmaceuticals and Medical Devices from a predetermined institution (hereinafter, simply referred to as a predetermined institution) in each country such as the Pharmaceuticals and Medical Devices Agency.

The medical institutions (research departments or the like that perform physician-led clinical trials, cohort studies, or the like), pharmaceutical companies, and CROs (departments or the like that perform marker searches, clinical trials, or the like) (hereinafter, these are collectively referred to as introduction sources) illustrated as an example in FIG. 18 correspond to, for example, the measurement system 10 in the above embodiment and trains the learner 100 using multimodal information collected in each of the above. That is, an introduction source is a party that generates the learned learner 100 and is an introduction source of the learned learner 100.

In each introduction source, multifaceted information (multimodal information) is collected using a wide variety of measuring devices 11, and the learner 100 is trained using the collected multimodal information, whereby the learned learner 100, in which the efficacy of a medicine depending on a case, the relationship with the prognosis, or others are learned, is generated. Therefore, learners 100 generated by respective introduction sources make use of the respective characteristics, in other words, go along with the purpose of research, development, and others of the respective introduction sources.

Furthermore, in each of the introduction sources, the degree of influence (contribution ratio) is calculated for each piece of modal information included in the multimodal information used for learning of the learner 100 as in the above-described embodiment. Each of the introduction sources narrows down modal information to be used for relearning of the learner 100 on the basis of the degree of influence of each piece of the modal information and relearns (also referred to as additional learning) the learner 100 using the narrowed-down modal information. As a result, the learner 100 is updated so that sufficient estimation accuracy can be achieved with the minimum necessary test (namely, acquisition of the minimum required modal information).

In a pharmaceutical approval application for the learned learner 100 (learned model 55), each of the introduction sources provides a predetermined institution with the pharmaceutical approval application and various types of information necessary therefor. The various types of information can include information for proving the effectiveness of the learned model 55 (estimation accuracy in a retrospective study, estimation accuracy in a prospective study, and others). Meanwhile, for example, in a case where the approval application reception system (which may include a part or all of processing from reception to approval) is automated by using cloud computing or the like, a server of each of the introduction sources (For example, this corresponds to the server 12 in the measurement system 10. Herein after referred to as the server 12.) may semi-automatically transmit an application for pharmaceutical approval and various types of information necessary for the application to a website for application for approval provided by the reception system. For example, the server 12 may provide a user with a tutorial for collecting various types of information necessary for a pharmaceutical approval application and automatically apply for pharmaceutical approval to the reception system using the various types of information collected thereby. At this point, the server 12 may automatically convert the collected various types of information into a format requested for the application.

The learner 100 for which a pharmaceutical approval has been obtained is introduced into a clinical site in a medical institution, for example. The clinical site may be, for example, medical setting where a rapid diagnostic test (RDT), a companion diagnosis, selection of external diagnosis medicine, and the like are performed and may be an introduction destination of the learner 100 for which the pharmaceutical approval has been obtained, the learner 100 having been provided by an introduction source. This clinical site can correspond to, for example, the measurement system 20 in the above embodiment.

Introduction of the learner 100 to a clinical site (hereinafter, also referred to as an introduction destination) may be in an aspect in which a part or all of the derivation device 40 incorporated with the learner 100 is installed in the clinical site or in an aspect in which a part or all of the derivation device 40 or the diagnosis assist system 1 incorporated with the learner 100 is on the cloud and provided to, for example, the server 22 in the clinical site as cloud service through a network.

In the clinical site where the learner 100 is introduced, a diagnostic result is estimated by inputting, to the learner 100, modal information obtained by diagnosis and test of a patient. At this point, an information acquiring device used to acquire the modal information from the patient may be an information acquiring device for acquiring modal information narrowed down by training in the introduction source. For example, in the example described using FIGS. 15 and 16 in the first embodiment, the pathological image necessary for estimation of a diagnosis result may be acquired and input to the learner 100 using only the digital pathology scanner among the digital pathology scanner for acquiring a pathological image and NGS for acquiring gene expression information.

Furthermore, in a clinical site, an estimation result of diagnosis acquired by the learner 100 may be compared with a reference based on data accumulated as clinical data. For example, the server 22 may display an estimation result (also referred to as a reference) obtained by inputting data (modal information) accumulated as clinical data to the learner 100 and an estimation result (diagnosis estimation result) obtained by inputting modal information obtained from a patient to be diagnosed to the learner 100 side by side to the user via the display device 24.

Note that the clinical site may be able to select a learner 100 to be used and to execute adjustment and the like of parameters (such as a weight w) of the learner 100 that has been selected for use. In addition, the clinical site may feed back or provide an estimation result of diagnosis obtained by the learner 100 or various types of modal information acquired by tests on patients of various other cases to research departments or the like of medical institutions, pharmaceutical companies, CROs, or others. For example, in a case where the accuracy of an estimation result (diagnosis estimation result) obtained by inputting modal information obtained from a patient to be diagnosed to the learner 100 is low (for example, in a case where the estimation accuracy is lower than that of a reference obtained from clinical data), modal information obtained from the patient to be diagnosed and a diagnosis result finally given by a doctor and the like to the patient may be fed back or provided to the introduction source as learning data (training data). Then, the fed-back or provided modal information may be used for cohort studies and the like in the introduction source.

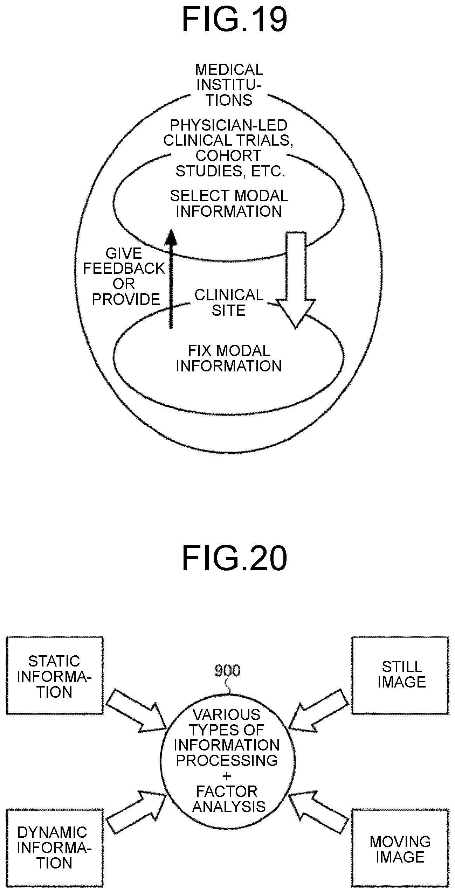

Note that, in the above description, a case where pharmaceutical promotion by a predetermined institution is required has been described as an example, however, it is not limited thereto, and for example, in a case where a medical institution adopts a laboratory developed test (LDT), training of the learner 100 to utilization in a clinical site may be completed in the medical institution as illustrated in FIG. 19. In this case, the pharmaceutical approval for the learner 100 may or may not be obtained.

4. Fourth Embodiment

In the above embodiments, cases where the learner in the diagnosis assist system using machine learning is trained by multimodal learning and used as a medical device have been described as an example, however, application examples of the technology according to the present disclosure are not limited to the diagnosis assist system and are not limited to the medical field.

That is, as illustrated in FIG. 20, in a learner 900 intended to enable automatic factor search using a wide variety of data such as static information, dynamic information, still images, and moving images, it is necessary to process a wide variety of information having different dimensions or different data formats, and thus there is a problem that no methodology for analysis is established. In addition, since it is necessary to handle an enormous amount of information at a time, there is also a problem that it is difficult to find information that has contributed to the estimation of the result.

The above embodiments are described as examples of means for solving these problems in the field of diagnosis assist. Therefore, in a fourth embodiment, cases where the technology according to the above embodiments (hereinafter, referred to as the technology of the disclosure) is applied to fields other than the field of diagnosis assist will be described with some examples.

4.1 Other Application Examples in Medical Field

First, another application example in the medical field will be described. As other application examples in the medical field, exemplary application to the field of precision medicine (hereinafter, referred to as the precision medicine field), exemplary application to the field of monitoring a treatment room such as an intensive care unit (ICU) or a high care unit (HCU) (hereinafter, referred to as the ICU monitoring field), and exemplary application to the field of improving efficiency of an operating room (hereinafter, referred to as the field of operating room efficiency improvement) will be described.

4.1.1 Exemplary Application to Precision Medicine Field

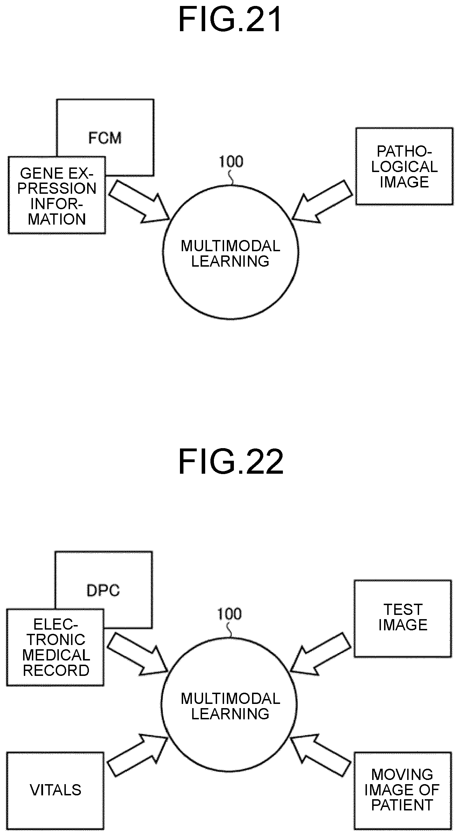

FIG. 21 is a diagram for explaining an application example of the technology according to the present disclosure to the precision medicine field. As illustrated in FIG. 21, in the precision medicine field, for example, the learner 100 performs multimodal learning using, as training data, multimodal information including gene expression information acquired by NGS, an analysis result of microparticles (cells and others) acquired by a flow cytometer (FCM), and a pathological image acquired by a digital pathology scanner. Then, at the time of actual diagnosis (at the time of inference) on a patient, modal information to be used or an information acquiring device for acquiring the modal information are narrowed down on the basis of the degree of influence of each piece of modal information calculated by the influence degree calculating unit 120, and modal information acquired by the information acquiring device that has been narrowed down or the modal information that has been narrowed down is input to the learned learner 100, thereby acquiring an inference result. As a result, in the precision medicine field, it is possible to grasp the general condition of a patient that is difficult to distinguish only by a pathological image, for example, and thus, it is possible to select a therapeutic agent or the like optimal for the condition of each patient (such as a tumor immunoresponse state).

4.1.2 Exemplary Application to ICU Monitoring Field

FIG. 22 is a diagram for explaining an application example of the technology according to the present disclosure to the ICU monitoring field. As illustrated in FIG. 22, in the ICU monitoring field, for example, the learner 100 performs multimodal learning using, as training data, multimodal information including an electronic medical record of a patient or points acquired in the diagnosis procedure combination system, vitals detected from the patient (such as the respiration, the body temperature, the blood pressure, or the pulse), a test image (such as a pathological image or a radiograph), and a moving image (patient moving image) obtained by imaging the patient in an ICU. Then, when monitoring the condition of the patient in the ICU, modal information to be used or an information acquiring device for acquiring the modal information are narrowed down on the basis of the degree of influence of each piece of modal information calculated by the influence degree calculating unit 120, and modal information acquired by the information acquiring device that has been narrowed down or the modal information that has been narrowed down is input to the learned learner 100, thereby acquiring an inference result. This makes it possible to grasp the condition of the patient, which is difficult to distinguish only by monitoring the vitals as in the related art, and thus, it is possible to present to the patient an optimal plan for improving the prognosis in addition to enabling optimal management of the general condition of each patient.

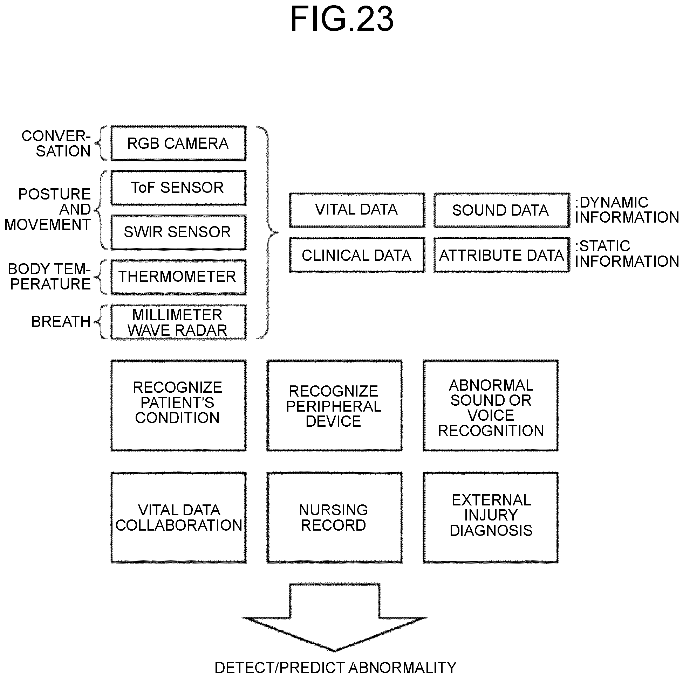

Incidentally, a specific example in a case where the technology according to disclosure is applied to the ICU monitoring field will be described by referring to FIG. 23. Note that FIG. 23 illustrates an example in which a condition risk biomarker search is performed on a patient in an ICU.

As illustrated in FIG. 23, in this example, sensor devices such as an RGB camera, a ToF sensor, a short wave infrared (SWIR) sensor, a thermometer, and a millimeter wave radar are arranged in the ICU as measuring devices 11. The RGB camera is used, for example, to detect facial expressions of the patient. The ToF sensor and the SWIR sensor are used, for example, to detect the posture or movement of the patient. thermometer is used, for example, to detect the body temperature of the patient, and the millimeter wave radar is used, for example, to detect the breaths of the patient. Note that, among the sensor data (corresponding to modal information) acquired by these sensor devices, vital data and audio data can be classified into dynamic information obtained by extracting information with a time width from the time series information, and clinical data or attribute data can be classified into static information like a snapshot obtained by extracting information at a certain moment.

Furthermore, in the present example, in addition to the sensor data (modal information) acquired by the measuring devices 11, a recognition result of the patient's condition, a recognition result of a peripheral device, a recognition result of an abnormal sound or voice, a result of association with the vital data, a nursing record, a diagnosis result of an external injury, and others are also acquired as modal information.

The learner 100 according to the above-described embodiments learns detection or prediction of an abnormality about the patient in the ICU by using these various types of modal information as input. Then, at the time of inference, by using modal information narrowed down at the time of learning as input, an abnormality of the patient in the ICU is detected or predicted. This makes it possible to reduce the number of pieces of sensor data to be acquired at the time of inference, thereby making it possible to improve the real-time property of detection or prediction of an abnormality by inference.

4.1.3 Exemplary Application to Field of Operating Room Efficiency Improvement

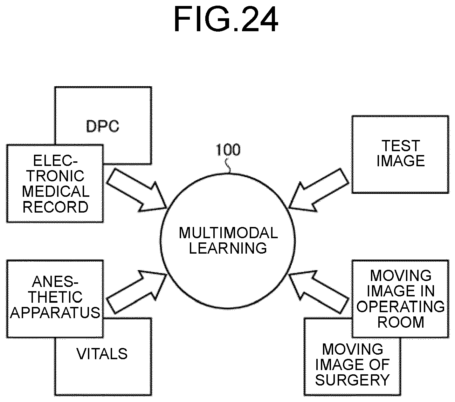

FIG. 24 is a diagram for explaining an application example of the technology according to the present disclosure to the field of operating room efficiency improvement. As illustrated in FIG. 24, in the field of operating room efficiency improvement, for example, the learner 100 performs multimodal learning using, as training data, multimodal information including an electronic medical record of a patient or points acquired in the diagnosis procedure combination system, vitals detected from the patient (such as the respiration, the body temperature, the blood pressure, or the pulse), a use situation of an anesthetic apparatus by an anesthesiologist, various test images (which may be a pathological image, a radiograph, or the like used for diagnosis), a moving image (moving image in the operating room) obtained by capturing the image of the entire operating room, and a moving image (moving image of the surgery) obtained by capturing the image of the state of the surgery. Then, at the time of improving the work efficiency during a surgery, modal information to be used or an information acquiring device for acquiring the modal information are narrowed down on the basis of the degree of influence of each piece of modal information calculated by the influence degree calculating unit 120, and modal information acquired by the information acquiring device that has been narrowed down or the modal information that has been narrowed down is input to the learned learner 100, thereby acquiring an inference result. This makes it possible to grasp, for example, a situation such as traveling or work of each member of the staff (including a doctor, a nurse, and others) that is difficult to distinguish only by the moving image in the operating room, and thus, it is possible to present an improved workflow for achieving efficiency improvement, safety improvement, and the like at the time of surgery.

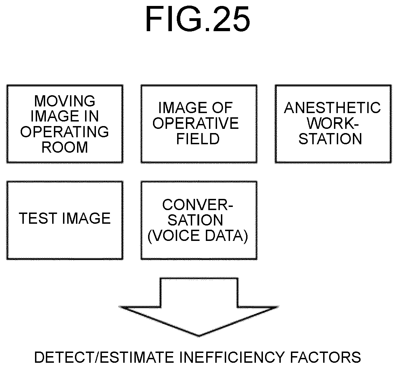

Incidentally, a specific example in a case where the technology according to disclosure is applied to the field of operating room efficiency improvement will be described by referring to FIG. 25. Note that FIG. 25 illustrates a case of searching for a factor of inefficiency of work in the operating room at the time of surgery.

As illustrated in FIG. 25, in the present example, a moving image in the operating room obtained by capturing an image of the entire operating room in a bird's eye view, a moving image of the operative field obtained by capturing an image of the state of the surgery, various types of information obtained from an anesthesia workstation that manages anesthesia for a patient at the time of the surgery, various types of information obtained by performing a test on the patient before the surgery, a conversation (voice data) of the doctor during the surgery, a scopist, or other medical staff, and the like are acquired as modal information.

The learner 100 according to the above-described embodiments learns factors of inefficiency of work in the operating room at the time of surgery by using these various types of modal information as input. Then, at the time of inference, by using modal information narrowed down at the time of learning as input, the factors of inefficiency of work in the operating room at the time of surgery are detected or estimated. This makes it possible to reduce the number of pieces of sensor data to be acquired at the time of inference, thereby making it possible to detect or predict the factors of inefficiency in real time.

4.2 Exemplary Application to Fields Other Than Medical Field

Next, application examples to fields other than the medical field will be described. As an exemplary application to a field other than the medical field, an exemplary application to the sports training field (by way of example, a golf swing) and an exemplary application to the smart agriculture field will be described.

4.2.1 Exemplary Application to Sports Training Field

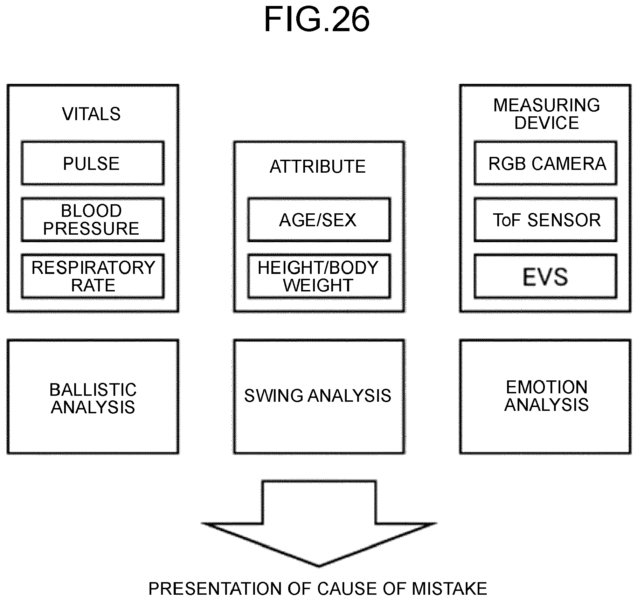

FIG. 26 is a diagram for explaining an application example of the technology according to the present disclosure to the field of sports training (as an example, a golf swing). As illustrated in FIG. 26, in the sports training field, the learner 100 performs multimodal learning using, as training data, multimodal information including, for example, vitals (the pulse, the blood pressure, the respiratory rate, etc.), attributes (age/sex, height/weight, etc.), a swing analysis result of a subject (such as a moving image of the swing (moving image acquired by an RGB camera) or the swing trajectory (data acquired by a ToF sensor, an EVS, an IMU, or the like)) of the subject, a ballistic analysis result (trajectory, initial speed/speed, rotation speed, and others) of the launched golf ball, an emotion analysis result, and others. Then, when giving advice on the golf swing, modal information to be used or an information acquiring device for acquiring the modal information are narrowed down on the basis of the degree of influence of each piece of modal information calculated by the influence degree calculating unit 120, and modal information acquired by the information acquiring device that has been narrowed down or the modal information that has been narrowed down is input to the learned learner 100, thereby acquiring an inference result (for example, an estimated cause of a mistake) as advice. As a result, for example, in a class where a golf lesson is given, it is possible to reduce equipment that needs to be provided, thereby making it possible to perform operation at low cost, to reduce the lesson fee, or others.

4.2.2 Exemplary Application to Smart Agriculture Field

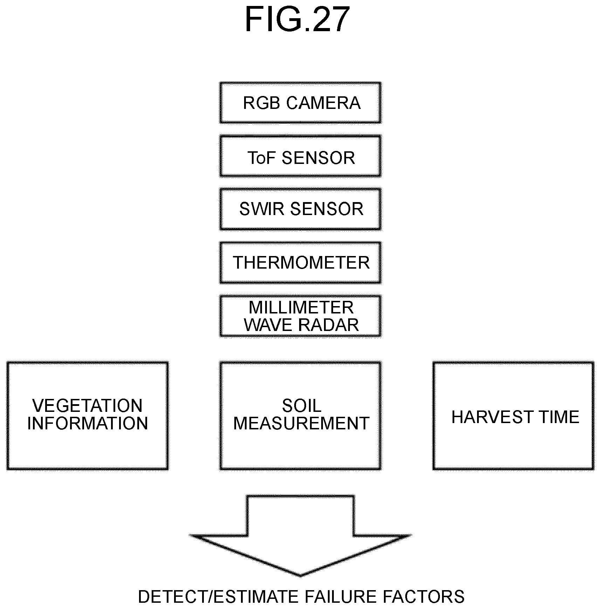

FIG. 27 is a diagram for explaining an application example of the technology according to the present disclosure to the smart agriculture field. As illustrated in FIG. 27, in the smart agriculture field, the learner 100 performs multimodal learning using, as training data, multimodal information including, for example, various types of information obtained by sensing farmland with an RGB camera, a ToF sensor, an SWIR sensor, a thermometer, or a millimeter wave radar, vegetation information obtained from these, a result of measuring the soil, harvest time predicted from measurement data such as an integrated thermometer, or the like. Then, when a factor of a failure is detected or predicted, modal information to be used or an information acquiring device for acquiring the modal information are narrowed down on the basis of the degree of influence of each piece of modal information calculated by the influence degree calculating unit 120, and modal information acquired by the information acquiring device that has been narrowed down or the modal information that has been narrowed down is input to the learned learner 100, thereby acquiring an inference result (for example, an estimated cause of the failure) as advice. As a result, for example, the number of measuring devices 11 to be installed in vast farmland can be reduced, thereby making it possible to reduce the initial cost or the running cost.

5. Hardware Configuration

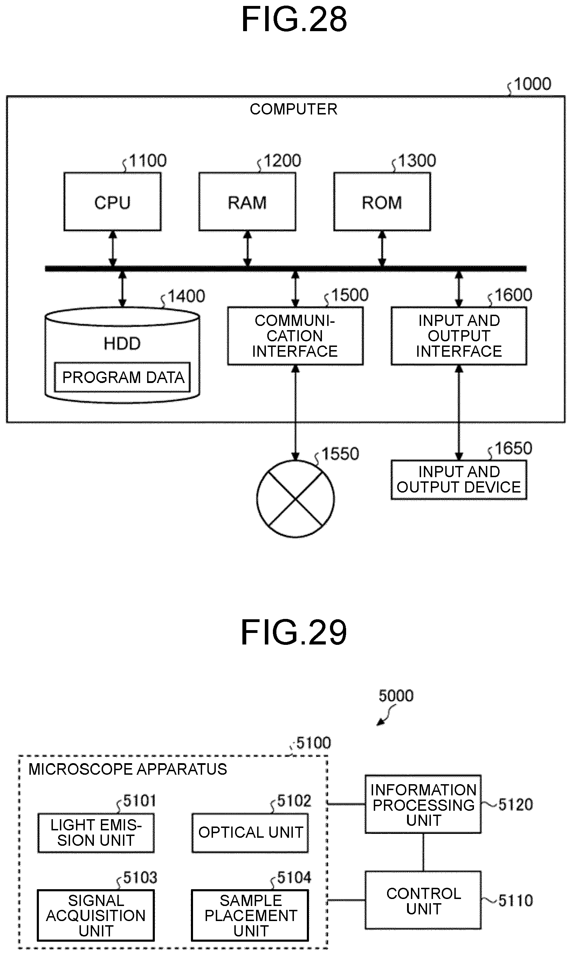

The server 12/22, the display control device 13/23, the medical information system 30, the derivation device 40, and others according to the embodiments and the modifications described above can be implemented by a computer 1000 having a configuration as illustrated in FIG. 28, for example. FIG. 28 is a hardware configuration diagram illustrating an example of the computer 1000 that implements functions of the server 12/22, the display control device 13/23, the medical information system 30, the derivation device 40, and others. The computer 1000 includes a CPU 1100, a R_AM 1200, a read only memory (ROM) 1300, a hard disk drive (HDD) 1400, a communication interface 1500, and an input and output interface 1600. The components of the computer 1000 are connected by a bus 1050.

The CPU 1100 operates in accordance with a program stored in the ROM 1300 or the HDD 1400 and controls each of the components. For example, the CPU 1100 loads a program stored in the ROM 1300 or the HDD 1400 in the RAM 1200 and executes processing corresponding to various programs.

The ROM 1300 stores a boot program such as a basic input output system (BIOS) executed by the CPU 1100 when the computer 1000 is activated, a program dependent on the hardware of the computer 1000, and the like.

The HDD 1400 is a computer-readable recording medium that non-transiently records a program to be executed by the CPU 1100, data used by such a program, and the like. Specifically, the HDD 1400 is a recording medium that records a program for executing operations according to the present disclosure, which is an example of program data 1450.

The communication interface 1500 is an interface for the computer 1000 to be connected with an external network 1550 (for example, the Internet). For example, the CPU 1100 receives data from another device or transmits data generated by the CPU 1100 to another device via the communication interface 1500.

The input and output interface 1600 includes the I/F unit 18 described above and is an interface for connecting an input and output device 1650 and the computer 1000. For example, the CPU 1100 receives data from an input device such as a keyboard or a mouse via the input and output interface 1600. In addition, the CPU 1100 transmits data to an output device such as a display, a speaker, or a printer via the input and output interface 1600. Furthermore, the input and output interface 1600 may function as a media interface that reads a program or the like recorded in a predetermined recording medium. A medium refers to, for example, an optical recording medium such as a digital versatile disc (DVD) or a phase change rewritable disk (PD), a magneto-optical recording medium such as a magneto-optical disk (MO), a tape medium, a magnetic recording medium, or a semiconductor memory.

For example, in a case where the computer 1000 functions as the server 12/22, the display control device 13/23, the medical information system 30, the derivation device 40, and others according to the above embodiments, the CPU 1100 of the computer 1000 implements the functions of the server 12/22, the display control device 13/23, the medical information system 30, the derivation device 40, and others by executing a program loaded on the RAM 1200. In addition, the HDD 1400 stores the program and others according to the present disclosure. Note that although the CPU 1100 reads the program data 1450 from the HDD 1400 and executes the program data 1450, as another example, these programs may be acquired from another device via the external network 1550.

6. Configuration Example of Microscope System of Present Disclosure

A configuration example of a microscope system of the present disclosure is illustrated in FIG. 29. A microscope system 5000 illustrated in FIG. 29 is an example of the measurement system 10/20 described above and includes, for example, a microscope apparatus 5100, a control unit 5110, and an information processing unit 5120. The microscope apparatus 5100 is an example of the measuring device 11/21 and includes, for example, a light emission unit 5101, an optical unit 5102, and a signal acquisition unit 5103. The microscope apparatus 5100 may further include a sample placement unit 5104 on which a biological sample S is placed. Note that the configuration of the microscope apparatus is not limited to that illustrated in FIG. 29, and for example, the light emission unit 5101 may be outside the microscope apparatus 5100, and for example, a light source not included in the microscope apparatus 5100 may be used as the light emission unit 5101. Furthermore, the light emission unit 5101 may be disposed in such a manner that the sample placement unit 5104 is interposed between the light emission unit 5101 and the optical unit 5102 and, for example, may be arranged on a side where the optical unit 5102 is present. The microscope apparatus 5100 may be capable of executing one or more of bright field observation, phase difference observation, differential interference observation, polarization observation, fluorescence observation, or dark field observation.

The microscope system 5000 may be configured as a so-called whole slide imaging (WSI) system or a digital pathology imaging system and may be used for pathological diagnosis. The microscope system 5000 may also be configured as a fluorescence imaging system, in particular a multiple fluorescence imaging system.

For example, the microscope system 5000 may be used to perform intraoperative pathological diagnosis or remote pathological diagnosis. In the intraoperative pathological diagnosis, while a surgery is being performed, the microscope apparatus 5100 can acquire data of the biological sample S acquired from a subject of the surgery and transmit the data to the information processing unit 5120. In the remote pathological diagnosis, the microscope apparatus 5100 can transmit the acquired data of the biological sample S to the information processing unit 5120 present in a remote place (such as in another room, or building) from the microscope apparatus 5100. Then, in these diagnoses, the information processing unit 5120 receives and outputs the data. A user of the information processing unit 5120 can perform pathological diagnosis on the basis of the output data.

(Biological Sample)

The biological sample S may contain a biological component. The biological component may be a tissue or a cell of a living body, a liquid component of a living body (blood, urine, etc.), culture, or a living cell (cardiomyocytes, nerve cells, a fertilized egg, etc.).

The biological sample may be a solid, a specimen fixed with a fixing reagent such as paraffin, or a solid formed by freezing. The biological sample may be a section of the solid. Specific examples of the biological sample include a section of a biopsy sample.

The biological sample may be subjected to treatment such as staining or labeling. The treatment may be staining for illustrating the form of the biological component or illustrating a substance (such as a surface antigen) of the biological component, and examples thereof include hematoxylin-eosin (HE) staining and immunohistochemistry staining. The biological sample may be subjected to the treatment with one or more reagents, and the reagent(s) may be fluorescent dye, a coloring reagent, a fluorescent protein, or a fluorescent-labeled antibody.

The specimen may be prepared from a tissue sample for the purpose of a pathological diagnosis, a clinical test, or the like. In addition, the specimen is not limited to a human body and may be derived from an animal, a plant, or other materials. The specimen has different properties depending on the type of tissue used (for example, organs or cells), the type of disease of concern, the attribute of the subject (e.g. age, sex, blood type, race, etc.), the lifestyle of the subject (e.g. dietary habits, exercise habits, smoking habits, etc.), or others. The specimen may be managed with identification information (a barcode, a QR code (registered trademark), or the like) that can identify each specimen.

(Light Emission Unit)

The light emission unit 5101 is a light source for illuminating the biological sample S and an optical unit that guides light emitted from the light source to a specimen. The light source may irradiate a biological sample with visible light, ultraviolet light, infrared light, or a combination thereof. The light source may be one or more of a halogen light source, a laser light source, an LED light source, a mercury light source, or a xenon light source. The type and/or wavelength of the light source in fluorescence observation may be plural and may be selected as appropriate by those skilled in the art. The light emission unit may have a transmissive, reflective, or epi-illumination (coaxial epi-illumination or side-illumination) configuration.

(Optical Unit)

The optical unit 5102 is configured so as to guide light from the biological sample S to the signal acquisition unit 5103. The optical unit can be configured so as to allow the microscope apparatus 5100 to observe or photograph the biological sample S.

The optical unit 5102 may include an objective lens. The type of the objective lens may be selected as appropriate by those skilled in the art depending on an observation system. The optical unit may also include a relay lens for relaying an image enlarged by the objective lens to the signal acquisition unit. The optical unit may further include an optical component other than the objective lens and the relay lens, an eyepiece lens, a phase plate, a condenser lens, and others.

Furthermore, the optical unit 5102 may further include a wavelength separation unit that separates light having a predetermined wavelength from the light from the biological sample S. The wavelength separation unit may selectively allow light of a predetermined wavelength or wavelength range to reach the signal acquisition unit. The wavelength separation unit may include, for example, one or more of a filter that selectively transmits light, a polarizing plate, a prism (Wollaston prism), or a diffraction grating. An optical component included in the wavelength separation unit may be disposed, for example, on an optical path from the objective lens to the signal acquisition unit. The wavelength separation unit is included in the microscope apparatus in a case where fluorescence observation is performed, particularly in a case where an excitation light emission unit is included. The wavelength separation unit may separate fluorescent light from each other or separate white light and fluorescent light.

(Signal Acquisition Unit)

The signal acquisition unit 5103 can receive light from the biological sample S and convert the light into an electric signal, particularly, a digital electric signal. The signal acquisition unit may be capable of acquiring data regarding the biological sample S on the basis of the electric signal. The signal acquisition unit may be capable of acquiring data of an image (an image, in particular, a still image, a time-lapse image or a moving image) of the biological sample S and, in particular, may acquire data of an image enlarged by the optical unit. The signal acquisition unit includes one or a plurality of imaging elements each including a plurality of pixels arranged one-dimensionally or two-dimensionally, a CMOS, a CCD, or the like. The signal acquisition unit may include an imaging element for acquiring a low-resolution image and an imaging element for acquiring a high-resolution image or may include an imaging element for sensing for AF or the like and an imaging element for image output for observation or the like. In addition to the plurality of pixels, the imaging element(s) may include a signal processing unit (one or more of a CPU, a DSP, or a memory) that performs signal processing using pixel signals from respective pixels and an output control unit that controls output of image data generated from the pixel signals and processing data generated by the signal processing unit. The imaging element including the plurality of pixels, the signal processing unit, and the output control unit can be preferably configured as a one-chip semiconductor device. Note that the microscope system 5000 may further include an event detection sensor. The event detection sensor may include a pixel that photoelectrically converts incident light and detect that a luminance change of the pixel exceeds a predetermined threshold value as an event. The event detection sensor may be particularly asynchronous.

(Control Unit)

The control unit 5110 controls imaging by the microscope apparatus 5100. The control unit can adjust the positional relationship between the optical unit and the sample placement unit by driving the movement of the optical unit 5102 and/or the sample placement unit 5104 for imaging control. The control unit 5110 can move the optical unit and/or the sample placement unit in a direction of approaching or separating from each other (for example, in the optical axis direction of the objective lens). The control unit may move the optical unit and/or the sample placement unit in any direction on a plane perpendicular to the optical axis direction. The control unit may control the light emission unit 5101 and/or the signal acquisition unit 5103 for imaging control.

(Sample Placement Unit)

The sample placement unit 5104 may be structured in such a manner that the position of a biological sample on the sample placement unit can be fixed and may be a so-called stage. The sample placement unit 5104 can be structured in such a manner that the position of the biological sample can be moved in the optical axis direction of the objective lens and/or a direction perpendicular to the optical axis direction.

(Information Processing Unit)

The information processing unit 5120 has a configuration corresponding to, for example, that of the server 12/22 and can acquire data (imaging data or others) acquired by the microscope apparatus 5100 from the microscope apparatus 5100. The information processing unit may execute image processing on the imaging data. The image processing may include unmixing processing, particularly, spectral unmixing processing. The unmixing processing may include processing of extracting data of light components of a predetermined wavelength or wavelength range from the imaging data to generate image data, processing of removing data of light components of a predetermined wavelength or wavelength range from the imaging data, or the like. In addition, the image processing may include autofluorescence separation processing of separating an autofluorescence component and a pigment component of a tissue section or fluorescence separation processing of separating wavelengths of pigments having different fluorescence wavelengths from each other. In the autofluorescence separation processing, processing may be performed in which an autofluorescence signal extracted from one of the plurality of specimens having the same or similar properties is used to remove an autofluorescence component from image information of the other specimen.

The information processing unit 5120 may transmit data for imaging control to the control unit 5110, and the control unit 5110 that has received the data may control imaging by the microscope apparatus 5100 in accordance with the data.

The information processing unit 5120 may be configured as an information processing device such as a general-purpose computer and may include a CPU, a RAM, and a ROM. The information processing unit may be included in a housing of the microscope apparatus 5100 or may be outside the housing. Furthermore, various types of processing or functions implemented by the information processing unit may be implemented by a server computer or a cloud connected via a network.

The imaging method of the biological sample S by the microscope apparatus 5100 may be selected as appropriate by those skilled in the art depending on the type of the biological sample, the purpose of imaging, or others. An example of the imaging method will be described below.

One example of the imaging method is as follows. The microscope apparatus may first identify an imaging target region. The imaging target region may be specified in such a manner that the entire region where the biological sample is present is covered or may be specified in such a manner that a target portion (a portion where a target tissue section, a target cell, or a target lesioned part is present) of the biological sample is covered. Next, the microscope apparatus divides the imaging target region into a plurality of divided regions of a predetermined size, and the microscope apparatus sequentially photographs each of the divided regions. As a result, an image of each of the divided regions is acquired.

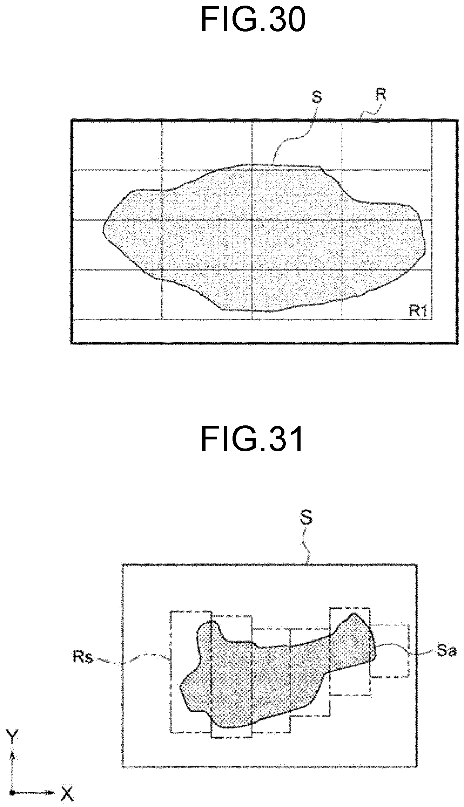

As illustrated in FIG. 30, the microscope apparatus specifies an imaging target region R covering the entire biological sample S. Then, the microscope apparatus divides the imaging target region R into sixteen divided regions. Then, the microscope apparatus may photograph a divided region R1 and then photograph any one of regions included in the imaging target region R, such as a region adjacent to the divided region R1. Then, the divided regions are photographed until there is no divided region that is not photographed. Note that a region other than the imaging target region R may also be photographed on the basis of captured image information of the divided regions.

In order to photograph a next divided region after photographing a certain divided region, the positional relationship between the microscope apparatus and the sample placement unit is adjusted. The adjustment may be performed by moving the microscope apparatus, moving the sample placement unit, or both of them. In this example, an imaging device that photographs each of the divided regions may be a two-dimensional imaging device (area sensor) or a one-dimensional imaging device (line sensor). The signal acquisition unit may photograph each of the divided regions via the optical unit. In addition, the photographing of each of the divided regions may be continuously performed while the microscope apparatus and/or the sample placement unit is/are moved, or moving the microscope apparatus and/or the sample placement unit may be stopped at the time of photographing each of the divided regions. The imaging target region may be divided in such a manner that parts of the divided regions overlap, or the imaging target region may be divided in such a manner that the divided regions do not overlap. Each of the divided regions may be photographed a plurality of times while imaging conditions such as a focal length and/or an exposure time are varied.

Furthermore, the information processing device can stitch a plurality of adjacent divided regions to generate image data of a wider region. By performing the stitching processing over the entire imaging target region, an image of a wider region can be acquired for the imaging target region. Moreover, image data with lower resolution can be generated from the images of the divided regions or the image obtained by the stitching processing.

Another example of the imaging method is as follows. The microscope apparatus may first identify an imaging target region. The imaging target region may be specified in such a manner that the entire region where the biological sample is present is covered or may be specified in such a manner that a target portion (a portion where a target tissue section or a target cell is present) of the biological sample is covered. Next, the microscope apparatus scans and photographs a partial region (also referred to as a "divided scan region") of the imaging target region in one direction (also referred to as a "scan direction") in a plane perpendicular to the optical axis. When the scan of the divided scan region is completed, next, a divided scan region adjacent to the scan region is scanned. These scanning operations are repeated until the entire imaging target region is photographed.

As illustrated in FIG. 31, the microscope apparatus specifies, as an imaging target region Sa, a region (gray portion) where a tissue section is present in the biological sample S. Then, the microscope apparatus scans a divided scan region Rs in the imaging target region Sa in the Y-axis direction. When the scan of the divided scan region Rs is completed, next, the microscope apparatus scans a divided scan region adjacent to the divided scan region in the X-axis direction. This operation is repeated until scanning is completed for the entire imaging target region Sa.

The positional relationship between the microscope apparatus and the sample placement unit is adjusted for scanning each of the divided scan regions and photographing a next divided scan region after photographing a certain divided scan region. The adjustment may be performed by moving the microscope apparatus, moving the sample placement unit, or both of them. In this example, an imaging device that photographs each of the divided scan regions may be a one-dimensional imaging device (line sensor) or a two-dimensional imaging device (area sensor). The signal acquisition unit may photograph each of the divided regions via a magnifying optical system. In addition, each of the divided scan regions may be continuously photographed while the microscope apparatus and/or the sample placement unit are/is moved. The imaging target region may be divided in such a manner that parts of the divided scan regions overlap, or the imaging target region may be divided in such a manner that the divided regions do not overlap. Each of the divided scan regions may be photographed a plurality of times while imaging conditions such as a focal length and/or an exposure time are varied.

Furthermore, the information processing device can stitch a plurality of adjacent divided scan regions to generate image data of a wider region. By performing the stitching processing over the entire imaging target region, an image of a wider region can be acquired for the imaging target region. Moreover, image data with lower resolution can be generated from the images of the divided scan regions or the image obtained by the stitching processing.

[Others]

Among the processing described in the above embodiments, the whole or a part of the processing described as that performed automatically can be performed manually, or the whole or a part of the processing described as that performed manually can be performed automatically by a known method. In addition, a processing procedure, a specific name, and information including various types of data or parameters illustrated in the above or in the drawings can be modified as desired unless otherwise specified. For example, various types of information illustrated in the drawings are not limited to the information illustrated.

In addition, each component of each device illustrated in the drawings is conceptual in terms of function and is not necessarily physically configured as illustrated in the drawings. That is, the specific form of distribution or integration of the devices is not limited to those illustrated in the drawings, and the whole or a part thereof can be functionally or physically distributed or integrated in any unit depending on various loads, usage status, and others.

In addition, the above embodiments and modifications can be combined as appropriate within a scope where there is no conflict in the processing content.

Note that the effects described herein are merely examples and are not limited, and other effects may also be achieved.

Note that the present technology can also have the following configurations.

(1) An information processing system including:

a machine learning model learned using learning data including two or more types of modal information acquired from a patient or a subject;
a setting unit that sets input data to be used as input to the machine learning model from the learning data; and
an output unit that inputs the input data to the machine learning model and outputs an estimation result of efficacy of a medicine or a health condition for the patient or the subject,
wherein the setting unit sets the input data on a basis of a degree of influence of each piece of the modal information included in the learning data on estimation of a diagnosis result at a time of learning of the machine learning model.

(2) The information processing system according to (1),
wherein the input data comprises at least one piece of modal information of a same type as a type of modal information included in the learning data.
(3) The information processing system according to (1) or (2),
wherein the input data comprises a number of types of modal information, the number being smaller than a number of types of the modal information included in the learning data.
(4) The information processing system according to any one of (1) to (3),
wherein the input data comprises modal information of a type different from the types of the modal information included in the learning data.
(5) The information processing system according to any one of (1) to (4), further including:
an information acquiring unit that acquires one or more pieces of modal information included in the input data.
(6) The information processing system according to (5),
wherein the information acquiring unit acquires, as the input data, modal information acquired by a smaller number of types of information acquiring devices than two or more types of information acquiring devices used for acquisition of each piece of the modal information included in the learning data.

(7) The information processing system according to (5),

wherein the information acquiring unit acquires, as the input data, modal information acquired by a same number of types of information acquiring devices as two or more types of information acquiring devices used for acquisition of each piece of the modal information included in the learning data, and
the setting unit selects modal information to be set as the input data from among two or more types of the modal information acquired by the information acquiring unit on a basis of the degree of influence.

(8) An information processing system including:

a generation unit that generates a machine learning model that outputs an estimation result of efficacy of a medicine or a health condition for a patient or a subject by using learning data including two or more types of modal information acquired from the patient or the subject; and
a calculation unit that calculates a degree of influence of each piece of the modal information included in the learning data on the estimation result of the efficacy of the medicine or the health condition at the time of generating the machine learning model,
wherein the generation unit changes the number of types of modal information included in the learning data to be used for generation of the machine learning model on a basis of the degree of influence.

(9) The information processing system according to (8),
wherein the generation unit sets, as the learning data, a predetermined number of pieces of modal information set in advance in descending order of values of the degrees of influence of the respective pieces of modal information.

(10) The information processing system according to (8) or (9),
wherein the generation unit sets, as the learning data, modal information of which degree of influence has a value equal to or greater than a preset threshold value among the degrees of influence of the respective pieces of modal information.

(11) The information processing system according to any one of (8) to (10),
wherein the generation unit excludes, from the learning data, modal information of which degree of influence is included in a preset ratio in an ascending order among the plurality of pieces of modal information.

(12) The information processing system according to any one of (8) to (11),
wherein the generation unit creates a management table that manages modal information of which input to the machine learning model is enabled and modal information of which input to the machine learning model is disabled among the plurality of pieces of modal information in a record for each piece of learning and manages, for each of records, estimation accuracy of the machine learning model when the machine learning model is learned by using the modal information enabled in each of the records of the management table.

(13) The information processing system according to any one of (8) to (12), further including:
an influence degree presenting unit that visualizes the influence degree of each piece of the modal information and presents the degree of influence to a user.

(14) The information processing system according to (13),
wherein the influence degree presenting unit visualizes and presents, to the user, a degree of influence of other modal information with respect to specific modal information in the two or more types of modal information included in the learning data.

(15) The information processing system according to (13),
wherein the influence degree presenting unit visualizes and presents to the user a region having affected derivation of the estimation result in at least one piece of modal information among the two or more types of modal information included in the learning data.

(16) The information processing system according to (13),

wherein meta-information is assigned to at least one of the two or more types of modal information included in the learning data, and
the influence degree presenting unit visualizes and presents to the user meta-information that has affected derivation of the estimation result among the meta-information assigned to the at least one piece of modal information.

(17) The information processing system according to any one of (13) to (16),
wherein the generation unit relearns the machine learning model using modal information selected by the user on a basis of the degree of influence presented by the influence degree presenting unit as the learning data.

(18) An information processing system including:

a generation unit that generates a machine learning model that outputs an estimation result of efficacy of a medicine or a health condition for a patient or a subject by using learning data including two or more types of modal information acquired from the patient or the subject;
a calculation unit that calculates a degree of influence of each piece of the modal information included in the learning data on an estimation result of the efficacy of the medicine or the health condition at the time of generation of the machine learning model;
a setting unit that sets input data as input to the machine learning model from the learning data on a basis of the degree of influence; and
an output unit that inputs the input data to the machine learning model and outputs an estimation result of efficacy of a medicine or a health condition for the patient or the subject,
wherein the generation unit changes the number of types of modal information included in the learning data to be used for generation of the machine learning model on a basis of the degree of influence, and
the setting unit sets the input data on a basis of a degree of influence of each piece of the modal information included in the learning data on estimation of a diagnosis result at the time of learning of the machine learning model.

(19) The information processing system according to any one of (1) to (18), further including:
a format conversion unit that converts a format of at least one piece of modal information among the plurality of pieces of modal information input to the machine learning model.
(20) The information processing system according to (19),
wherein the format conversion unit converts at least one piece of modal information among the plurality of pieces of modal information into image data of a fixed size.
(21) The information processing system according to any one of (1) to (20),
wherein the estimation result of the efficacy of the medicine or the health condition includes at least one of a health condition of the patient or the subject or efficacy of a medicine prescribed for the patient or the subject.
(22) The information processing system according to any one of (1) to (21),
wherein the plurality of pieces of modal information includes at least one of a pathological image, gene expression information, blood protein information, a radiograph, medical history information, an antibody test result, intestinal bacterial flora information, or lifestyle information.
(23) The information processing system according to (22),
wherein the lifestyle information includes at least one of hours of sleep, a heart rate, a number of steps, a blood oxygen concentration, or a blood glucose level.
(24) An information processing method including the steps of:

setting input data to be used as input to a machine learning model learned using learning data including two or more types of modal information acquired from a patient or a subject from among the learning data; and
inputting the input data to the machine learning model and outputting an estimation result of efficacy of a medicine or a health condition for the patient or the subject,
wherein the input data is set on a basis of a degree of influence given to estimation of a diagnosis result at a time of learning of the machine learning model, the degree of influence given by each piece of the modal information included in the learning data.

(25) An information processing method including the steps of:

generating a machine learning model that outputs an estimation result of efficacy of a medicine or a health condition for a patient or a subject by using learning data including two or more types of modal information acquired from the patient or the subject; and
calculating a degree of influence of each piece of the modal information included in the learning data on the estimation result of the efficacy of the medicine or the health condition at the time of generating the machine learning model,
wherein the number of types of modal information included in the learning data to be used for generation of the machine learning model is changed on a basis of the degree of influence.

(26) An information processing method including the steps of:

generating a machine learning model that outputs an estimation result of efficacy of a medicine or a health

# EP 4 307 311 A1

condition for a patient or a subject by using learning data including two or more types of modal information acquired from the patient or the subject;

calculating a degree of influence of each piece of the modal information included in the learning data on an estimation result of the efficacy of the medicine or the health condition at the time of generation of the machine learning model;

setting input data as input to the machine learning model from the learning data on a basis of the degree of influence; and

inputting the input data to the machine learning model and outputting an estimation result of efficacy of a medicine or a health condition for the patient or the subject,

wherein the number of types of modal information included in the learning data to be used for generation of the machine learning model is changed on a basis of the degree of influence, and

the input data is set on a basis of a degree of influence of each piece of the modal information included in the learning data on estimation of a diagnosis result at the time of learning of the machine learning model.

Reference Signs List

1 DIAGNOSIS ASSIST SYSTEM
10, 20 MEASUREMENT SYSTEM
11, 21 MEASURING DEVICE
11A MEASURING DEVICE GROUP
12, 22 SERVER
13, 23 DISPLAY CONTROL DEVICE
14, 24 DISPLAY DEVICE
18 I/F UNIT
30 MEDICAL INFORMATION SYSTEM
40 DERIVATION DEVICE
41 COMMUNICATION UNIT
42 STORAGE UNIT
43 CONTROL UNIT
51 MEASUREMENT INFORMATION ACQUIRING UNIT
51A INFORMATION ACQUIRING UNIT GROUP
52 DIAGNOSTIC INFORMATION ACQUIRING UNIT
53 LEARNING UNIT
55 LEARNED MODEL
56 SELECTOR
100, 100A, 200 LEARNER
101, 101a to 101n NEURAL NETWORK UNIT
102 CONCATENATION OR ADDITION UNIT
103 AFFINE AND SOFTMAX UNIT
104, 104a to 104n GMP UNIT
120 INFLUENCE DEGREE CALCULATING UNIT
201, 201a to 201n FORMAT CONVERSION UNIT
5000 MICROSCOPE SYSTEM
5100 MICROSCOPE APPARATUS
5101 LIGHT EMISSION UNIT
5102 OPTICAL UNIT
5103 SIGNAL ACQUISITION UNIT
5104 SAMPLE PLACEMENT UNIT
5110 CONTROL UNIT
5120 INFORMATION PROCESSING UNIT

**Claims**

**1.** An information processing system including:

a machine learning model learned using learning data including two or more types of modal information acquired from a patient or a subject;

a setting unit that sets input data to be used as input to the machine learning model from the learning data; and

an output unit that inputs the input data to the machine learning model and outputs an estimation result of efficacy of a medicine or a health condition for the patient or the subject,

wherein the setting unit sets the input data on a basis of a degree of influence of each piece of the modal information included in the learning data on estimation of a diagnosis result at a time of learning of the machine learning model.

2. The information processing system according to claim 1,
wherein the input data comprises at least one piece of modal information of a same type as a type of modal information included in the learning data.

3. The information processing system according to claim 1,
wherein the input data comprises a number of types of modal information, the number being smaller than a number of types of the modal information included in the learning data.

4. The information processing system according to claim 1,
wherein the input data comprises modal information of a type different from the types of the modal information included in the learning data.

5. The information processing system according to claim 1, further including:
an information acquiring unit that acquires one or more pieces of modal information included in the input data.

6. The information processing system according to claim 5,
wherein the information acquiring unit acquires, as the input data, modal information acquired by a smaller number of types of information acquiring devices than two or more types of information acquiring devices used for acquisition of each piece of the modal information included in the learning data.

7. The information processing system according to claim 5,

wherein the information acquiring unit acquires, as the input data, modal information acquired by a same number of types of information acquiring devices as two or more types of information acquiring devices used for acquisition of each piece of the modal information included in the learning data, and

the setting unit selects modal information to be set as the input data from among two or more types of the modal information acquired by the information acquiring unit on a basis of the degree of influence.

8. An information processing system including:

a generation unit that generates a machine learning model that outputs an estimation result of efficacy of a medicine or a health condition for a patient or a subject by using learning data including two or more types of modal information acquired from the patient or the subject; and

a calculation unit that calculates a degree of influence of each piece of the modal information included in the learning data on the estimation result of the efficacy of the medicine or the health condition at the time of generating the machine learning model,

wherein the generation unit changes the number of types of modal information included in the learning data to be used for generation of the machine learning model on a basis of the degree of influence.

9. The information processing system according to claim 8,
wherein the generation unit sets, as the learning data, a predetermined number of pieces of modal information set in advance in descending order of values of the degrees of influence of the respective pieces of modal information.

10. The information processing system according to claim 8,
wherein the generation unit sets, as the learning data, modal information of which degree of influence has a value equal to or greater than a preset threshold value among the degrees of influence of the respective pieces of modal information.

11. The information processing system according to claim 8,

wherein the generation unit excludes, from the learning data, modal information of which degree of influence is included in a preset ratio in an ascending order among the plurality of pieces of modal information.

12. The information processing system according to claim 8,
wherein the generation unit creates a management table that manages modal information of which input to the machine learning model is enabled and modal information of which input to the machine learning model is disabled among the plurality of pieces of modal information in a record for each piece of learning and manages, for each of records, estimation accuracy of the machine learning model when the machine learning model is learned by using the modal information enabled in each of the records of the management table.

13. The information processing system according to claim 8, further including:
an influence degree presenting unit that visualizes the influence degree of each piece of the modal information and presents the degree of influence to a user.

14. The information processing system according to claim 13,
wherein the influence degree presenting unit visualizes and presents, to the user, a degree of influence of other modal information with respect to specific modal information in the two or more types of modal information included in the learning data.

15. The information processing system according to claim 13,
wherein the influence degree presenting unit visualizes and presents to the user a region having affected derivation of the estimation result in at least one piece of modal information among the two or more types of modal information included in the learning data.

16. The information processing system according to claim 13,

wherein meta-information is assigned to at least one of the two or more types of modal information included in the learning data, and
the influence degree presenting unit visualizes and presents to the user meta-information that has affected derivation of the estimation result among the meta-information assigned to the at least one piece of modal information.

17. The information processing system according to claim 13,
wherein the generation unit relearns the machine learning model using modal information selected by the user on a basis of the degree of influence presented by the influence degree presenting unit as the learning data.

18. An information processing system including:

a generation unit that generates a machine learning model that outputs an estimation result of efficacy of a medicine or a health condition for a patient or a subject by using learning data including two or more types of modal information acquired from the patient or the subject;
a calculation unit that calculates a degree of influence of each piece of the modal information included in the learning data on an estimation result of the efficacy of the medicine or the health condition at the time of generation of the machine learning model;
a setting unit that sets input data as input to the machine learning model from the learning data on a basis of the degree of influence; and
an output unit that inputs the input data to the machine learning model and outputs an estimation result of efficacy of a medicine or a health condition for the patient or the subject,
wherein the generation unit changes the number of types of modal information included in the learning data to be used for generation of the machine learning model on a basis of the degree of influence, and
the setting unit sets the input data on a basis of a degree of influence of each piece of the modal information included in the learning data on estimation of a diagnosis result at the time of learning of the machine learning model.

19. The information processing system according to claim 1, further including:
a format conversion unit that converts a format of at least one piece of modal information among the plurality of pieces of modal information input to the machine learning model.

20. The information processing system according to claim 19,
wherein the format conversion unit converts at least one piece of modal information among the plurality of pieces of modal information into image data of a fixed size.

21. The information processing system according to claim 1,
wherein the estimation result of the efficacy of the medicine or the health condition includes at least one of a health condition of the patient or the subject or efficacy of a medicine prescribed for the patient or the subject.

22. The information processing system according to claim 1,
wherein the plurality of pieces of modal information includes at least one of a pathological image, gene expression information, blood protein information, a radiograph, medical history information, an antibody test result, intestinal bacterial flora information, or lifestyle information.

23. The information processing system according to claim 22,
wherein the lifestyle information includes at least one of hours of sleep, a heart rate, a number of steps, a blood oxygen concentration, or a blood glucose level.

24. An information processing method including the steps of:

setting input data to be used as input to a machine learning model learned using learning data including two or more types of modal information acquired from a patient or a subject from among the learning data; and
inputting the input data to the machine learning model and outputting an estimation result of efficacy of a medicine or a health condition for the patient or the subject,
wherein the input data is set on a basis of a degree of influence given to estimation of a diagnosis result at a time of learning of the machine learning model, the degree of influence given by each piece of the modal information included in the learning data.

25. An information processing method including the steps of:

generating a machine learning model that outputs an estimation result of efficacy of a medicine or a health condition for a patient or a subject by using learning data including two or more types of modal information acquired from the patient or the subject; and
calculating a degree of influence of each piece of the modal information included in the learning data on the estimation result of the efficacy of the medicine or the health condition at the time of generating the machine learning model,
wherein the number of types of modal information included in the learning data to be used for generation of the machine learning model is changed on a basis of the degree of influence.

26. An information processing method including the steps of:

generating a machine learning model that outputs an estimation result of efficacy of a medicine or a health condition for a patient or a subject by using learning data including two or more types of modal information acquired from the patient or the subject;
calculating a degree of influence of each piece of the modal information included in the learning data on an estimation result of the efficacy of the medicine or the health condition at the time of generation of the machine learning model;
setting input data as input to the machine learning model from the learning data on a basis of the degree of influence; and
inputting the input data to the machine learning model and outputting an estimation result of efficacy of a medicine or a health condition for the patient or the subject,
wherein the number of types of modal information included in the learning data to be used for generation of the machine learning model is changed on a basis of the degree of influence, and
the input data is set on a basis of a degree of influence of each piece of the modal information included in the learning data on estimation of a diagnosis result at the time of learning of the machine learning model.

EP 4 307 311 A1

# FIG.1

32

# FIG.2

DERIVATION DEVICE — 40

COMMUNICATION UNIT — 41

CONTROL UNIT — 43

MEASUREMENT INFORMATION ACQUIRING UNIT — 51

DIAGNOSTIC INFORMATION ACQUIRING UNIT — 52

LEARNING UNIT — 53

STORAGE UNIT — 42

LEARNED MODEL — 55

EP 4 307 311 A1

# FIG.3

EP 4 307 311 A1

# FIG.4

EP 4 307 311 A1

# FIG.5

| MODAL 1 | MODAL 2 | MODAL 3 | ••• | MODAL n | ESTIMATION ACCURACY |
|---------|---------|---------|-----|---------|---------------------|
| 1 | 0 | 1 | ••• | 0 | 0.87 |
| 1 | 1 | 0 | ••• | 1 | 0.54 |
| 0 | 1 | 1 | ••• | 1 | 0.63 |
| 1 | 0 | 1 | ••• | 0 | 0.94 |
| ⋮ | ⋮ | ⋮ | ⋮ | ⋮ | ⋮ |

# FIG.6

START

N=1 — S101

SET VALID OR INVALID TO
EACH MODAL IN DATA SET — S102

LEARN LEARNER USING DATA SET — S103

EVALUATE LEARNED LEARNER — S104

N=N+1 — S105

NO

$N > N\_max$ ? — S106

YES

CALCULATE DEGREE OF
INFLUENCE (CONTRIBUTION RATIO) OF
EACH MODAL IN DATA SET — S107

OUTPUT CALCULATED DEGREE OF
INFLUENCE (CONTRIBUTION RATIO) OF
EACH MODAL — S108

END

# FIG.7

DEGREE OF INFLUENCE

| | | |
|---|---|---|
| 0.5 | MODAL #1 | → CNN (101a) |
| -0.2 | MODAL #2 | → CNN (101b) |
| 0.1 | MODAL #3 | → RNN (101c) |
| 0.2 | MODAL #n | → Transformer (101n) |

LEARNER (100)

Concatenate or Add (102)

Affine + Softmax (103)

OUTPUT

EP 4 307 311 A1

# FIG.8

EP 4 307 311 A1

FIG.9

|  | PATHOLOGICAL IMAGE | GENE EXPRESSION INFORMATION | BLOOD PROTEIN INFORMATION | RADIOGRAPH | MEDICAL HISTORY INFORMATION | ANTIBODY TEST RESULT | INTESTINAL BACTERIAL FLORA INFORMATION | LIFESTYLE INFORMATION |
|---|---|---|---|---|---|---|---|---|
| PATHOLOGICAL IMAGE | — | | | | | | | |
| GENE EXPRESSION INFORMATION | | — | | | | | | |
| BLOOD PROTEIN INFORMATION | | | — | | | | | |
| RADIOGRAPH | | | | — | | | | |
| MEDICAL HISTORY INFORMATION | | | | | — | | | |
| ANTIBODY TEST RESULT | | | | | | — | | |
| INTESTINAL BACTERIAL FLORA INFORMATION | | | | | | | — | |
| LIFESTYLE INFORMATION | | | | | | | | — |

ADVERSE INFLUENCE          FAVORABLE INFLUENCE

40

# FIG.10

ADVERSE
INFLUENCE

FAVORABLE
INFLUENCE

# FIG.11

ADVERSE INFLUENCE    FAVORABLE INFLUENCE

EP 4 307 311 A1

# FIG.12

FIG.13

# FIG.14

# FIG.15

EP 4 307 311 A1

# FIG.16

# FIG.17

EP 4 307 311 A1

# FIG.18

# FIG.19

# FIG.20

# FIG.21

# FIG.22

# FIG.23

CONVER-SATION { RGB CAMERA

POSTURE AND MOVEMENT { ToF SENSOR / SWIR SENSOR

BODY TEM-PERATURE { THERMOMETER

BREATH { MILLIMETER WAVE RADAR

| VITAL DATA | SOUND DATA | :DYNAMIC INFORMATION |
| CLINICAL DATA | ATTRIBUTE DATA | :STATIC INFORMATION |

| RECOGNIZE PATIENT'S CONDITION | RECOGNIZE PERIPHERAL DEVICE | ABNORMAL SOUND OR VOICE RECOGNITION |
| VITAL DATA COLLABORATION | NURSING RECORD | EXTERNAL INJURY DIAGNOSIS |

DETECT/PREDICT ABNORMALITY

52

# FIG.24

# FIG.25

| | | |
|---|---|---|
| MOVING IMAGE IN OPERATING ROOM | IMAGE OF OPERATIVE FIELD | ANESTHETIC WORK-STATION |
| TEST IMAGE | CONVER-SATION (VOICE DATA) | |

DETECT/ESTIMATE INEFFICIENCY FACTORS

# FIG.26

| | | |
|---|---|---|
| **VITALS** | | **MEASURING DEVICE** |
| PULSE | **ATTRIBUTE** | RGB CAMERA |
| BLOOD PRESSURE | AGE/SEX | ToF SENSOR |
| RESPIRATORY RATE | HEIGHT/BODY WEIGHT | EVS |
| BALLISTIC ANALYSIS | SWING ANALYSIS | EMOTION ANALYSIS |

PRESENTATION OF CAUSE OF MISTAKE

# FIG.27

RGB CAMERA

ToF SENSOR

SWIR SENSOR

THERMOMETER

MILLIMETER
WAVE RADAR

| VEGETATION INFORMATION | SOIL MEASUREMENT | HARVEST TIME |

DETECT/ESTIMATE FAILURE FACTORS

# FIG.28

COMPUTER 〔1000

CPU 〔1100

RAM 〔1200

ROM 〔1300

HDD 〔1400
PROGRAM DATA

COMMUNI-CATION INTERFACE 〔1500

INPUT AND OUTPUT INTERFACE 〔1600

〔1550

INPUT AND OUTPUT DEVICE 〔1650

# FIG.29

5000

MICROSCOPE APPARATUS 〔5100

LIGHT EMIS-SION UNIT 〔5101

OPTICAL UNIT 〔5102

SIGNAL ACQUISITION UNIT 〔5103

SAMPLE PLACEMENT UNIT 〔5104

INFORMATION PROCESSING UNIT ~5120

CONTROL UNIT ~5110

# FIG.30

# FIG.31

## INTERNATIONAL SEARCH REPORT

International application No.

**PCT/JP2022/007706**

### A. CLASSIFICATION OF SUBJECT MATTER

*G16H 20/00*(2018.01)i; *G16H 50/20*(2018.01)i; *G06N 20/00*(2019.01)i
FI: G16H50/20; G16H20/00; G06N20/00 130

According to International Patent Classification (IPC) or to both national classification and IPC

### B. FIELDS SEARCHED

Minimum documentation searched (classification system followed by classification symbols)

G16H10/00-99/00; G06Q10/00-99/00; G06N20/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2006-172461 A (ADEZA BIOMEDICAL CORP) 29 June 2006 (2006-06-29) paragraphs [0037], [0077]-[0087] | 1-7, 19-24 |
| Y | | 8-11, 18, 25, 26 |
| A | | 12-17 |
| Y | WO 2020/138085 A1 (KYOCERA CORP) 02 July 2020 (2020-07-02) paragraphs [0001], [0049], [0050] | 8-11, 18, 25, 26 |
| A | JP 2003-6329 A (HITACHI LTD) 10 January 2003 (2003-01-10) entire text, all drawings | 1-26 |
| A | WO 2021/039339 A1 (CANON KK) 04 March 2021 (2021-03-04) entire text, all drawings | 1-26 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **25 March 2022** | **05 April 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT
### Information on patent family members

| | International application No. |
|---|---|
| | **PCT/JP2022/007706** |

| Patent document cited in search report | | | Publication date (day/month/year) | Patent family member(s) | Publication date (day/month/year) |
|---|---|---|---|---|---|
| JP | 2006-172461 | A | 29 June 2006 | US 2001/0023419 A1 paragraphs [0039], [0116]-[0128] | |
| WO | 2020/138085 | A1 | 02 July 2020 | US 2022/082574 A1 paragraphs [0001], [0053], [0054] | |
| JP | 2003-6329 | A | 10 January 2003 | (Family: none) | |
| WO | 2021/039339 | A1 | 04 March 2021 | CN 114207736 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020174863 A

**Non-patent literature cited in the description**

- **SATOSHI HARA ; ATSUSHI NITANDA ; TAKA-NORI MAEHARA.** Data Cleaning for Models Trained with SGD. *arXiv: 1906.08473 (2019), NIPS,* 2019

- **PANG WEI KOH ; PERCY LIANG.** Understanding Black-box Predictions via Influence Functions. *Stanford University, ICML,* 2017
- **COOK, R. DENNIS ; SANFORD WEISBERG.** Residuals and Influence in Regression. Chapman and Hall, 1982